(19) **Europäisches Patentamt / European Patent Office / Office européen des brevets**

(11) **EP 4 167 129 B1**

(12) **EUROPEAN PATENT SPECIFICATION**

(45) Date of publication and mention
of the grant of the patent:
**09.07.2025  Bulletin 2025/28**

(21) Application number: **22770210.7**

(22) Date of filing: **27.01.2022**

(51) International Patent Classification (IPC):
**A61B 5/08** *(2006.01)*   **A61B 5/11** *(2006.01)*
**A61B 5/296** *(2021.01)*   **A61B 5/397** *(2021.01)*
**G06F 3/01** *(2006.01)*   **G06F 1/16** *(2006.01)*
**A61B 5/113** *(2006.01)*   **A61B 5/1455** *(2006.01)*
**A61B 5/318** *(2021.01)*   **G06F 17/18** *(2006.01)*

(52) Cooperative Patent Classification (CPC):
**G06F 3/011; A61B 5/0022; A61B 5/0816;
A61B 5/1116; A61B 5/1121; A61B 5/1123;
A61B 5/296; A61B 5/397; A61B 5/6804;
A61B 5/7207; A61B 5/7246; A61B 5/725;
A61B 5/7253; A61B 5/7275; A61B 5/7278;**  (Cont.)

(86) International application number:
**PCT/CN2022/074379**

(87) International publication number:
**WO 2022/193851 (22.09.2022 Gazette 2022/38)**

(54) **METHOD AND SYSTEM FOR RECOGNIZING USER ACTIONS**

VERFAHREN UND SYSTEM ZUR ERKENNUNG VON BENUTZERAKTIONEN

PROCÉDÉ ET SYSTÈME DE RECONNAISSANCE D'ACTIONS D'UTILISATEUR

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority:  **19.03.2021  PCT/CN2021/081931**

(43) Date of publication of application:
**19.04.2023  Bulletin 2023/16**

(73) Proprietor: **Shenzhen Shokz Co., Ltd.
Shenzhen, Guangdong 518108 (CN)**

(72) Inventors:
• **LI, Meiqi
  Shenzhen, Guangdong 518000 (CN)**
• **SU, Lei
  Shenzhen, Guangdong 518000 (CN)**

• **ZHOU, Xin
  Shenzhen, Guangdong 518000 (CN)**
• **LIAO, Fengyun
  Shenzhen, Guangdong 518000 (CN)**
• **QI, Xin
  Shenzhen, Guangdong 518000 (CN)**

(74) Representative: **Wang, Bo
Panovision IP
Ebersberger Straße 3
85570 Markt Schwaben (DE)**

(56) References cited:
CN-A- 106 073 793   CN-A- 107 349 594
CN-A- 107 349 594   CN-A- 108 209 910
CN-A- 108 211 309   CN-A- 110 569 775
CN-A- 110 569 775   US-A1- 2020 302 236

(Cont. next page)

(52) Cooperative Patent Classification (CPC): (Cont.)
 **G06F 1/163; G06F 1/1684; G06F 3/015;**
 **G06F 3/017;** A61B 5/113; A61B 5/14551;
 A61B 5/318; A61B 5/6803; A61B 5/7257;
 A61B 5/726; A61B 5/7405; A61B 5/7455;
 A61B 2505/09; A61B 2562/0219; G06F 17/18;
 G06F 2218/08

**Description**

**TECHNICAL FIELD**

The present disclosure relates to the technical field of wearable apparatus, and in particular, to methods and systems for identifying a user action.

**BACKGROUND**

With people's attention to scientific sports and physical health, fitness motion monitoring device is developing greatly. At present, the main way for the motion monitoring device to monitor the user's action is to analyze the user's action data based on the reference action data when the type of the user's action is known, so as to monitor whether the user's action is standardized. Therefore, in a practical application scenario, the user needs to inform the motion monitoring device of the fitness action type in advance before exercising, so that the motion monitoring device can select the reference action data of the action type to make accurate monitoring of the user action. For the user, before each fitness action, the action type must be informed to the motion monitoring device, which leads to a poor user experience. Moreover, the existing motion monitoring devices monitor the user's action in a non-real time manner, which results in that the user can only receive information related to the fitness action after completing the fitness action, which also leads to a poor user experience.

Therefore, it is necessary to provide a method and a system for identifying a fitness action of a user in real-time without the need for the user to input the action type in advance.

Methods and systems according to the state of the art are for instance described in CN108211309A, CN108209910A, CN110569775A and CN107349594A.

**SUMMARY**

The present invention concerns a method of identifying a user action, comprising: obtaining user action data collected from a plurality of measurement positions on a user, the user action data corresponding to an unknown user action; identifying, based on at least one set of target reference action data, that the user action includes a target action when obtaining the user action data, the at least one set of target reference action data corresponding to the target action, wherein the identifying that the user action includes a target action comprises: obtaining a plurality of sets of candidate reference action data, wherein each set of candidate reference action data corresponds to at least one reference action; performing a two-level screening operation on the plurality of sets of candidate reference action data based on the user action data, the two-level screening operation including a combination of a difference degree-based screening operation and a probability-based screening operation, including: obtaining a plurality of sets of reference action data, wherein each set of reference action data corresponds to at least one reference action; selecting each set of reference action data in turn from the plurality of sets of reference action data as candidate reference action data; determining at least one difference degree by comparing at least one segment of action identification sub-data of the candidate reference action data with the corresponding user action sub-data segment by segment; and determining a comprehensive difference degree by weighting and summing the at least one difference degree; selecting N pieces of second-level candidate reference action data from the plurality of sets of reference action data, a value of the comprehensive difference degree of the second-level candidate reference action data being less than a first preset value, and N being an integer greater than 1; calculating N distances between the user action data and the N pieces of second-level candidate reference action data respectively; calculating N probability values based on the N distances respectively; selecting the second-level candidate reference action data whose probability value is greater than a second preset value as the target reference action data; and determining a reference action corresponding to the target reference action data as the target action; and determining that the user action includes the target action based on a result of the two-level screening operation; and sending information related to the target action to the user.

In some embodiments, each set of reference action data may include M pieces of reference action sub-data, each piece of the reference action sub-data may include at least one segment of action identification sub-data, and M may be an integer greater than 1. Action identification sub-data of the M pieces of reference action sub-data may form integral action identification data, and each segment of action identification sub-data may correspond to at least a portion of the reference action on at least one measurement position of the plurality of measurement positions.

In some embodiments, the determining at least one difference degree by comparing at least one segment of action identification sub-data of the candidate reference action data with the corresponding user action sub-data segment by segment may include the following operations. A sliding window with a preset length on each piece of the action identification sub-data may be selected, the sliding window may include a data segment of the user action data collected in a preset time interval. For the sliding window at a current moment, the difference degree between the data segment and the corresponding action identification sub-data may be determined.

In some embodiments, the identifying that the user action includes the target action further may include the following operations. A value of the comprehensive difference degree is greater than a first preset value may be determined. The sliding window may slide to a next data segment with a preset step size, and the comparison may be repeated.

In some embodiments, a data collection time length corresponding to the data segment in the sliding window may be negatively correlated with a user action speed.

In some embodiments, the preset step size may satisfy one or more following conditions. The preset step size may be positively correlated with a magnitude of a value of the comprehensive difference degree at a previous moment. The preset step size may be positively correlated with a variation trend of the value of the comprehensive difference degree.

In some embodiments, the data segment may include a plurality of user action data points. The determining at least one difference degree by comparing at least one segment of action identification sub-data of the candidate reference action data with the corresponding user action sub-data segment by segment may include the following operations. A target comparison data interval may be selected from the action identification sub-data, wherein the target comparison data interval includes a plurality of identification data points. The data segment according to a plurality of scales may be adjusted to obtain a plurality of adjusted data segments. A difference degree between the action identification sub-data and each adjusted data segment of the plurality of adjusted data segments may be determined respectively. A minimum difference degree between the action identification sub-data and the data segment may be determined.

In some embodiments, the determining at least one difference degree by comparing at least one segment of action identification sub-data of the candidate reference action data with the corresponding user action sub-data segment by segment may include the following operations. A distance matrix $[D_{ij}]$ may be determined, wherein $D_{ij}$ denotes a distance between an i-th data point of a target comparison data interval and a j-th data point of the data segment. A shortest distance path of the distance matrix may be determined, wherein the shortest distance path may satisfy the following operations. A start point of the shortest distance path may be in the first line of the $[D_{ij}]$, two adjacent points on the shortest distance path may be adjacent in the distance matrix, a next point on the shortest distance path may be to the right, below or right below a previous point, an end point of the shortest distance path may be in a last line of the $[D_{ij}]$, the shortest distance path may have a smallest regularization cost, wherein the regularization cost is determined by distances of points on the corresponding shortest distance path of the distance matrix, and he difference degree may be related to the regularization cost.

In some embodiments, if the first data point of the data segment may be determined to be a data point where the user action starts, the start point of the shortest distance path may be a distance $D_{11}$ between the first point of the data segment and the first point of the target comparison data interval.

In some embodiments, if the last data point of the data segment may be determined to be the data point where the user action ends, the end point of the shortest distance path may be a distance $D_{mn}$ between the last point of the data segment and the last point of the target comparison data interval.

The invention further concerns a system and computer readable medium according to claims 11 and 12, respectively.

## BRIEF DESCRIPTION OF THE DRAWINGS

The present disclosure may be further described by way of exemplary embodiments, which may be described in detail by means of the accompanying drawings. These embodiments are not limiting, and in these embodiments, the same numbers refer to the same structures, wherein:

FIG. 1 illustrates a schematic diagram of an application scenario of a motion monitoring system according to some embodiments of the present disclosure;

FIG. 2 illustrates a schematic diagram of exemplary hardware and/or software components of a wearable apparatus according to some embodiments of the present disclosure;

FIG. 3 illustrates a schematic diagram of exemplary hardware and/or software components of a computing device according to some embodiments of the present disclosure;

FIG. 4 illustrates an exemplary structural diagram of a wearable apparatus according to some embodiments of the present disclosure;

FIG. 5 is a flowchart illustrating an exemplary process for determining a target action according to some embodiments of the present disclosure;

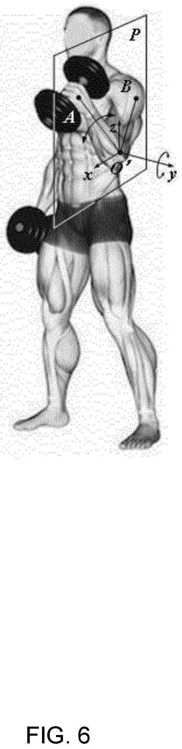

FIG. 6 illustrates an exemplary coordinate system diagram during a user's motion according to some embodiments of the present application;

FIG. 7A shows an exemplary segment of action identification data in reference action data and a curve of a segment of the user action sub-data collected by the sliding window in the user action data on the time axis according to some embodiments of the present disclosure;

FIG. 7B illustrates a distance matrix and a shortest distance path from the upper left corner to the lower right corner of the distance matrix according to some embodiments of the present disclosure;

FIG. 7C illustrates a schematic diagram of determining the comprehensive difference degree through a sliding window when the user action data includes a plurality of user action sub-data according to some embodiments of the present disclosure.

## DETAILED DESCRIPTION

In order to illustrate technical solutions of the embodiments of the present disclosure more clearly, the following briefly illustrates drawings in the illustration of the embodiments. Drawings in the following illustration are merely some examples or embodiments of the present disclosure. For those skilled in the art, the present disclosure may be applied to other similar scenarios in accordance with the drawings without creative works. Unless obviously obtained from the context or the context illustrates otherwise, the same number in the drawings refers to the same structure or operation.

It should be understood that "system," "device," "unit," and/or "module" used herein are a method for distinguishing different components, elements, members, portions, or assemblies of different levels. However, if other words may achieve the same purpose, the words may be replaced by other expressions.

As used in the disclosure and the appended claims, the singular forms "a," "an," and "the" include plural referents unless the content clearly dictates otherwise. In general, the terms "comprising" and "including" only prompt steps and elements that are explicitly identified, and these steps and elements do not constitute an exclusive list. Methods or device may also include other steps or elements.

Flowcharts are used in the present disclosure to illustrate the operations performed by the system according to some embodiments of the present disclosure. It should be understood that the front or rear operations may not be necessarily performed exactly in order. On the contrary, each step may be performed in reverse or simultaneously. At the same time, other operations may also be added to the procedures, or a certain step or several steps may be removed from the procedures.

The present disclosure provides a target action determination system. A set of instructions stored in a storage medium in the target action determination system may be executed to obtain user action data during the user's motion. The target action determination system may be applied to a wearable apparatus (e.g., a clothing, a wristband, and a helmet), a medical testing device (e.g., an electromyographic (EMG) tester), a fitness device, etc. After the user wears the device, sensors on the device may be attached to a plurality of measurement positions on the user's body, so the user's action data may be collected by the sensors on the device. After the sensors collect the user action data, the processor in the target action determination system may communicate with the storage medium to access or read the instructions stored in the storage medium, etc. When the target action determination system is running, the processor may access reference action data stored in the storage medium with known action content. Based on the reference action data of these known action contents, the system may perform a target action identification on the user action data whose action contents are unknown. After determining the target action, the system may send a content related to the target action to the user.

In some embodiments of the present disclosure, the system may perform the target action identification on the user action data immediately or within a predetermined time, the predetermined time may be a short time, such as 0.1 seconds or 0.5 seconds. In this way, the system may realize real-time identification of the user action data, and the user may immediately receive the related content about the action after performing the action.

In some embodiments of the present disclosure, the user action data may also be obtained in other ways without being collected by sensors on devices such as a wearable apparatus (e.g., a clothing, a wristband, a helmet), a medical detection device (e.g., an EMG tester), a fitness device, etc. For example, user images in a video may be analyzed by an artificial intelligence algorithm to obtain action data of several measurement positions on the user's body. In a word, as long as the user action data may be obtained in real time, the method and system of the present disclosure may be configured to determine the target action.

The storage medium may include a propagated data signal having a computer program code embodied therein, for example, at baseband or as part of a carrier wave. The propagated signal may take various manifestations, including electromagnetic, optical, etc., or a suitable combination. The computer storage medium may be any computer-readable medium other than computer-readable storage medium that may communicate, propagate, or transmit a program for use by coupling to an instruction execution system, an apparatus, or a device. The program code on the computer storage medium may be transmitted over any suitable medium, including radio, a cable, a fiber optic cable, RF, or the like, or a combination thereof. Specifically, the storage medium may be a random-access memory (RAM), a read only memory (ROM), etc. Exemplary ROM may include a mask ROM (MROM), a programmable ROM (PROM), an erasable programmable ROM (PEROM), an electrically erasable programmable ROM (EEPROM), a compact disc ROM (CD-ROM), a digital universal disc ROM, etc. Exemplary RAM may include a dynamic RAM (DRAM), a double rate synchronous dynamic RAM (DDR SDRAM), a static RAM (SRAM), a thyristor RAM (T-RAM), a zero capacitance (Z-RAM), or the like.

As an example, the processor may be a multi-core processor, a single-core processor, a distributed processor, a central processing unit (CPU), an application-specific integrated circuit (ASIC), an application-specific instruction processor (ASIP), a graphics processor (GPU), a physical processor (PPU), a digital signal processor (DSP), a field programmable gate array (FPGA), a programmable logic circuit (PLD), a controller, a microcontroller unit, a reduced instruction set computer (RISC), a microprocessor device, or the like, or any combination thereof.

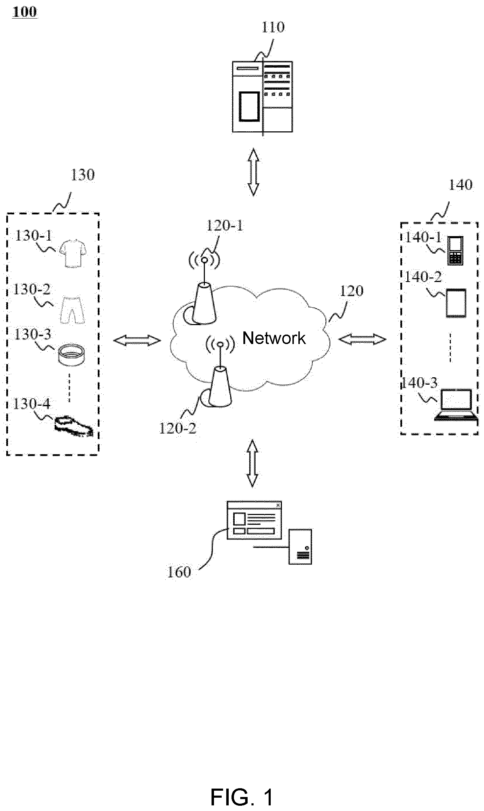

FIG. 1 illustrates a schematic diagram of an application scenario of a motion monitoring system according to some embodiments of the present disclosure. As shown in FIG. 1, the system 100 for determining a target action (or system 100) may include a processing device 110, a network 120, a wearable apparatus 130, and a mobile terminal device 140. The system 100 may obtain user action data (e.g., an EMG signal, a posture signal, stress data, and physiological monitoring data such as an ECG signal, a respiratory rate signal, etc.) configured to characterize the user's action, and identify which target action the action of the user belongs to when exercising according to the user action data.

For example, the system 100 may identify an action of a user performed by the user while exercising. When the user wears the wearable apparatus 130 for fitness exercise, the wearable apparatus 130 may obtain the user's action data. The processing device 110 or the mobile terminal device 140 may receive and analyze the user's action data to identify the user's fitness action, such as whether the user's fitness action is a bench press, a bicep curl, or a squat, etc., so as to send a content related to the target action to the user, wherein the user action of the identified action (e.g., a bench press, a bicep curl, a squat, etc.) is the target action. Specifically, the content related to the target action may include the name of the target action prompted by voice or video, the action type of the action, the action quantity, the action time, the user's physiological parameter information when the user performs the action, etc. Further, the system 100 may generate feedback on the user's fitness action based on the analysis result of the user's fitness action data, such as whether the user's fitness action is standard, etc., so as to guide the user's fitness.

As another example, the system 100 may identify the user's action performed by the user while running. For example, when the user wears the wearable apparatus 130 to perform a running motion, the system 100 may obtain the user's running action data and identify that the user's current motion is running based on reference action data. When the user runs for too long or the running action is incorrect, the fitness device may feed back his/her motion status to the user to prompt the user to adjust the running action or running time.

In some embodiments, the processing device 110 may be configured to process information and/or data related to user motion. For example, the processing device 110 may receive the user's action signal (e.g., an EMG signal, a posture signal, an ECG signal, a respiratory rate signal, etc.), and further extract feature information (e.g., the feature information corresponding to the EMG signal or the feature information corresponding to the posture signal in the action signal) corresponding to the action signal. In some embodiments, the processing device 110 may perform specific signal processing on the EMG signal or gesture signal collected by the wearable apparatus 130, such as signal segmentation, signal preprocessing (e.g., signal correction processing, filtering processing, etc.), or the like. In some embodiments, the processing device 110 may also determine whether the user's action is correct based on the user's action signal. For example, the processing device 110 may determine whether the user's action is correct based on the feature information (e.g., amplitude information, frequency information, etc.) corresponding to the EMG signal. As another example, the processing device 110 may determine whether the user's action is correct based on feature information (e.g., angular velocity, angular velocity direction, angular velocity acceleration, angle, displacement information, stress, etc.) corresponding to the gesture signal. As further another example, the processing device 110 may determine whether the user's action is correct based on the feature information corresponding to the EMG signal and the feature information corresponding to the gesture signal. In some embodiments, the processing device 110 may also determine whether the user's physiological parameter information during the user's motion meets the health standard. In some embodiments, the processing device 110 may also issue corresponding instructions to feed back the user's motion situation. For example, when the user is running, the system 100 monitors that the user's running time is too long. At this time, the processing device 110 may issue an instruction to the mobile terminal device 140 to prompt the user to adjust the running time. It should be noted that the feature information corresponding to the gesture signal is not limited to the above-mentioned angular velocity, angular velocity direction, angular velocity acceleration, angle, displacement information, stress, etc., but may also be other feature information. Any parameter information that can reflect the relative motion of the user's body may be the feature information corresponding to the gesture signal. For example, when the posture sensor is a strain gauge sensor, a bending angle and a bending direction of the user's joint may be obtained by measuring the magnitude of the resistance in the strain gauge sensor that changes with the stretched length.

In some embodiments, the processing device 110 may be local or remote. For example, the processing device 110 may access information and/or data stored in the wearable apparatus 130 and/or the mobile terminal device 140 through the network 120. In some embodiments, the processing device 110 may connect directly with the wearable apparatus 130 and/or the mobile terminal device 140 to access information and/or data stored therein. For example, the processing device 110 may be located in the wearable apparatus 130 and realize information interaction with the mobile terminal device 140 through the network 120. As another example, the processing device 110 may be located in the mobile terminal device 140 and realize information interaction with the wearable apparatus 130 through the network. In some embodiments, the processing device 110 may be implemented on a cloud platform.

In some embodiments, the processing device 110 may process data and/or information related to action monitoring to perform one or more functions described herein. In some embodiments, the processing device 110 may obtain an action signal during the user's motion collected by the wearable apparatus 130. In some embodiments, the processing device 110 may send a control instruction to the wearable apparatus 130 or the mobile terminal device 140. The control instruction may control the states of switches of the wearable apparatus 130 and sensors of the wearable apparatus 130, and may also control the mobile terminal device 140 to send out prompt information. In some embodiments, the processing device 110 may include one or more sub-processing devices (e.g., a single-core processing device or a multi-core processing device).

The network 120 may facilitate the exchange of data and/or information of the motion monitoring system 100. In some embodiments, one or more components of the motion monitoring system 100 may send data and/or information to other components of the motion monitoring system 100 through network 120. For example, an action signal collected by the wearable apparatus 130 may be transmitted to the processing device 110 through the network 120. As another example, a confirmation result for the action signal determined by the processing device 110 may be transmitted to the mobile terminal device 140 through the network 120. In some embodiments, the network 120 may be any type of wired or wireless network.

The wearable apparatus 130 may refer to a garment or apparatus with a wearable function. In some embodiments, the wearable apparatus 130 may include, but is not limited to, an upper garment device 130-1, a pants device 130-2, a wristband device 130-3, a shoe device 130-4, or the like. In some embodiments, the wearable apparatus 130 may include M sensors, M is an integer greater than one. The sensors may obtain various action signals (e.g., an EMG signal, a posture signal, temperature information, a heartbeat frequency, an electrocardiogram signal, etc.) generated during the user's motion. In some embodiments, the sensors may include, but are not limited to, one or more of an EMG sensor, a posture sensor, a temperature sensor, a humidity sensor, an electrocardiogram sensor, a blood oxygen saturation sensor, a Hall sensor, an electrodermal sensor, a rotation sensor, or the like. For example, the upper garment device 130-1 may include an EMG sensor positioned at a muscle (e.g., biceps brachii, triceps brachii, latissimus dorsi, trapezius, etc.) position of the human body, and the EMG sensor may fit the user's skin and collect an EMG signal during the user's motion. As another example, the upper garment device 130-1 may include an electrocardiogram sensor positioned near the left pectoral muscle of the human body, and the electrocardiogram sensor may collect an electrocardiographic signal of the user. As further another example, the pants device 130-2 may include a posture sensor positioned at a muscle (e.g., gluteus maximus, vastus lateralis, vastus medialis, gastrocnemius, etc.) position of the human body, and the posture sensor may collect a posture signal of the user. In some embodiments, the wearable apparatus 130 may also provide feedback on the user's action. For example, when an action of a certain portion of the body during the user's motion does not meet the standard, the EMG sensor corresponding to this portion may generate a stimulation signal (e.g., current stimulation or hitting signal) to remind the user.

It should be noted that the wearable apparatus 130 is not limited to the upper garment device 130-1, the pants device 130-2, the wristband device 130-3, or the shoe device 130-4 shown in FIG.1. The wearable apparatus 130 may also include other apparatuses used for motion monitoring, such as a helmet device, a kneepad device, etc., which is not limited here. Any apparatus that can use the motion monitoring method disclosed in the present disclosure is within the scope of protection of the present disclosure.

In some embodiments, the mobile terminal device 140 may obtain information or data in the system 100. In some embodiments, the mobile terminal device 140 may receive action data processed by the processing device 110, and feed back an action record based on the processed action data. Exemplary feedback modes may include, but are not limited to, a voice prompt, an image prompt, a video presentation, a text prompt, or the like. In some embodiments, the user may obtain the action record during his/her own motion through the mobile terminal device 140. For example, the mobile terminal device 140 may be connected with the wearable apparatus 130 through the network 120 (e.g., wired connection, wireless connection). The user may obtain the action record during the user's motion through the mobile terminal device 140, and the action record may be transmitted to the processing device 110 through the mobile terminal device 140. In some embodiments, the mobile terminal device 140 may include a mobile device 140-1, a tablet computer 140-2, a notebook computer 140-3, or the like, or any combination thereof. In some embodiments, the mobile device 140-1 may include a cell phone, a smart home device, a smart mobile device, a virtual reality device, an augmented reality device, etc., or any combination thereof. In some embodiments, the smart home device may include a control device for a smart appliance, a smart monitoring device, a smart TV, a smart camera, etc., or any combination thereof. In some embodiments, the smart mobile device may include a smart phone, a personal digital assistant (PDA), a gaming device, a navigation device, a POS device, etc., or any combination thereof. In some embodiments, the virtual reality device and/or the augmented reality device may include a virtual reality headset, virtual reality glasses, a virtual reality eyewear, an augmented reality helmet, augmented reality glasses, an augmented reality eyewear, etc., or any combination thereof.

In some embodiments, the motion monitoring system 100 may also include an action data presentation system 160. The action data presentation system 160 may be configured to process and display information and/or data related to the user's action. For example, what kind of motion the user is doing may be displayed, or the information and/or data may be combined with a virtual character and intuitively displayed on a user interface of the mobile terminal device 140 to facilitate the user to view. For example, the action data presentation system 160 may receive the user's action data. For instance, the user's action data may include an action signal such as an EMG signal, a posture signal, an electrocardiogram signal, a respiratory rate signal, etc. As another example, the user's action data may include feature information (e.g., feature information corresponding to the EMG signal, feature information corresponding to the gesture signal in the action signal) obtained by the processing device 110 performing feature processing on the action signal. As further another example, the user's action data may include a single obtained after the processing device 110 performs specific signal processing, such as signal segmentation, signal preprocessing (e.g., signal correction processing, filtering processing, etc.), etc. The action data presentation system 160 may compare the action data with the reference action data, combine the comparison result with a virtual character to generate an animation of the virtual character, and send the generated animation to the mobile terminal device 140 for display. The reference action data may be described in detail in the following descriptions. For example, when the user is doing biceps curling, the action data presentation system 160 may receive the action data of the user when performing biceps curling, such as an EMG signal of the biceps brachii, an EMG signal of the trapezius, a movement posture of the forearm, a movement posture of the forearm, etc. The action data presentation system 160 may compare the user's action data with a plurality of sets of reference action data stored in the motion monitoring system 100 to determine that the user is performing the action of biceps curling. Further, the action data presentation system 160 may display a virtual character that is doing biceps curling, and the user may clearly and intuitively view the user's action data or a difference between the action data and the reference action data (e.g., a difference in the position and size of muscle forces, a difference in the action posture, etc.) through the animation of the virtual character to adjust the action during the motion.

In some embodiments, the action data presentation system 160 may be integrated in the processing device 110. In some embodiments, the action data presentation system 160 may also be integrated in the mobile terminal device 140. In some embodiments, the action data presentation system 160 may also exist independently of the processing device 110 and the mobile terminal device 140. The action data presentation system 160 may be connected in communication with the processing device 110, the wearable apparatus 130, and the mobile terminal device 140 to transmit and exchange information and/or data. In some embodiments, the action data presentation system 160 may access information and/or data stored in the processing device 110, the wearable apparatus 130, and/or the mobile terminal device 140 via the network 120. In some embodiments, the wearable apparatus 130 may connect directly with the processing device 110 and/or mobile terminal device 140 to access information and/or data stored therein. For example, the action data presentation system 160 may be located in the processing device 110 and realize information interaction with the wearable apparatus 130 and the mobile terminal device 140 through the network 120. As another example, the action data presentation system 160 may be located in the mobile terminal device 140 and realize information interaction with the processing device 110 and the wearable apparatus 130 through the network. In some embodiments, the action data presentation system 160 may be executed on a cloud platform, and realize information interaction with the processing device 110, the wearable apparatus 130, and the mobile terminal device 140 through the network.

For the convenience of presentation, in the following descriptions, the action data presentation system 160 located in the mobile terminal device 140 may be taken as an example to the description.

In some embodiments, the action data presentation system 160 may process data and/or information related to action data presentation to perform one or more functions described herein. In some embodiments, the action data presentation system 160 may obtain action data during the user's motion, for example, an action signal during the user's motion collected by the wearable apparatus 130, or data obtained after the action signal collected during the user's motion by the wearable device 130 is processed by the processing device 110. In some embodiments, the action data presentation system 160 may send a control instruction to the mobile terminal device 140 to control the display of the user interface of the mobile terminal device 140.

In some embodiments, the system 100 may also include a database. The database may store data (e.g., an initially preset threshold condition, etc.) and/or instructions (e.g., a feedback instruction). In some embodiments, the database may store data obtained from the wearable apparatus 130 and/or the mobile terminal device 140. In some embodiments, the database may store information and/or instructions for execution or use by the processing device 110 to perform the exemplary methods described in the present disclosure. In some embodiments, the database may be connected with the network 120 to communicate with one or more components of the system 100 (e.g., the processing device 110, the wearable apparatus 130, the mobile terminal device 140, etc.). One or more components of the system 100 may access data or instructions stored in the database through the network 120. In some embodiments, the database may connect or communicate directly with one or more components in the system 100. In some embodiments, the database may be portion of the processing device 110.

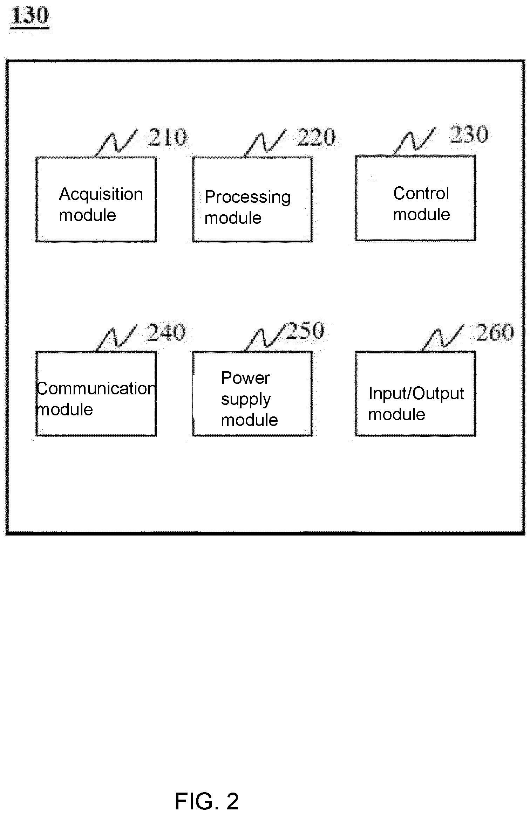

FIG. 2 illustrates a schematic diagram of exemplary hardware and/or software components of a wearable apparatus according to some embodiments of the present disclosure. As shown in FIG. 2, the wearable apparatus 130 may include an acquisition module 210, a processing module 220 (also referred to as a processor), a control module 230 (also referred to as a main controller, an MCU, a controller), a communication module 240, a power supply module 250, and an input/output module 260.

The acquisition module 210 may be configured to obtain an action signal during a motion of a user. In some embodiments, the acquisition module 210 may include a sensor unit, and the sensor unit may be configured to obtain one or more action signals during the user's motion. In some embodiments, the sensor unit may include, but is not limited to one or more of an EMG sensor, a posture sensor, an electrocardiogram sensor, a respiration sensor, a temperature sensor, a humidity sensor, an inertial sensor, a blood oxygen saturation sensor, a Hall sensor, an electrodermal sensor, a rotation sensor, or the like. In some embodiments, the action signal may include one or more of an EMG signal, a posture signal, an electrocardiogram signal, a respiratory rate, a temperature signal, a humidity signal, or the like. The sensor unit may be placed in different positions of the wearable apparatus 130 according to the type of the action signal to be obtained. For example, in some embodiments, the EMG sensor (also referred to as an electrode element) may be disposed at a position of a human muscle, and the EMG sensor may be configured to collect an EMG signal during the user's motion. The EMG signal and the corresponding feature information (e.g., frequency information, amplitude information, etc.) thereof may reflect a state of the muscle during the user's motion. The gesture sensor may be set at different positions of the human body (e.g., positions corresponding to the trunk, limbs, and joints in the wearable apparatus 130), and the gesture sensor may be configured to collect the gesture signal during the user's motion. The gesture signal and the corresponding feature information (e.g., an angular velocity direction, an angular velocity value, an angular velocity acceleration value, an angle, displacement information, a stress, etc.) thereof may reflect the gesture of the user's motion. The ECG sensor may be arranged at a position around the chest of the human body, and the ECG sensor may be configured to collect the ECG data during the user's motion. The respiration sensor may be arranged at a position around the chest of the human body, and the respiration sensor may be configured to collect respiration data (e.g., a respiration frequency, a respiration amplitude, etc.) during the user's motion. The temperature sensor may be configured to collect temperature data (e.g., a body surface temperature) during the user's motion. The humidity sensor may be configured to collect humidity data of the external environment during the user's motion.

The processing module 220 may process data from the acquisition module 210, the control module 230, the communication module 240, the power supply module 250, and/or the input/output module 260. For example, the processing module 220 may process the action signal during the user's motion from the acquisition module 210. In some embodiments, the processing module 220 may preprocess the action signal (e.g., an EMG signal, a gesture signal) obtained by the acquisition module 210. For example, the processing module 220 may perform segmentation processing on the EMG signal or the gesture signal during the user's motion. As another example, the processing module 220 may perform preprocessing (e.g., filtering processing, signal correction processing) on the EMG signal during the user's motion to improve the quality of the EMG signal. As further another example, the processing module 220 may determine feature information corresponding to the gesture signal based on the gesture signal during the user's motion. In some embodiments, the processing module 220 may process an instruction or an operation from the input/output module 260. In some embodiments, the processed data may be stored in a memory or a hard disk. In some embodiments, the processing module 220 may transmit the processed data to one or more components of the motion monitoring system 100 via the communication module 240 or the network 120. For example, the processing module 220 may send a monitoring result of the user's motion to the control module 230, and the control module 230 may execute subsequent operations or instructions according to an action determined result.

The control module 230 may be connected with other modules of the wearable apparatus 130. In some embodiments, the control module 230 may control operating states of other modules of the wearable apparatus 130. For example, the control module 230 may control a power supply state (e.g., a normal mode, a power saving mode), a power supply time, etc., of the power supply module 250. As another example, the control module 230 may control the input/output module 260 according to the user's action determined result, so as to control the mobile terminal device 140 to send a feedback result of the user's motion to the user. If there is a problem with the action (e.g., the action does not meet the standard) of the user during the user's motion, the control module 230 may control the input/output module 260, so as to control the mobile terminal device 140 to give feedback to the user, so that the user may know his/her own motion state in real-time and adjust the action. In some embodiments, the control module 230 may also control one or more sensors of the acquisition module 210 or other modules to provide feedback to the human body. For example, if a certain muscle exerts too much force during the user's motion, the control module 230 may control an electrode module at the position of the muscle to electrically stimulate the user to prompt the user to adjust the action in time.

In some embodiments, the communication module 240 may be configured to exchange information or data. In some embodiments, the communication module 240 may be configured for communication between components of the wearable apparatus 130. For example, the acquisition module 210 may send a user action signal (e.g., an EMG signal, a gesture signal, etc.) to the communication module 240, and the communication module 240 may send the action signal to the processing module 220. In some embodiments, the communication module 240 may also be configured for communication between the wearable apparatus 130 and other components in the system 100. For example, the communication module 240 may send state information (e.g., a switch state) of the wearable apparatus 130 to the processing device 110, and the processing device 110 may monitor the wearable apparatus 130 based on the state information. The communication module 240 may adopt wired, wireless, and wired/wireless hybrid technologies.

In some embodiments, the power supply module 250 may provide power to other components in the system 100.

The input/output module 260 may obtain, transmit, and send a signal. The input/output module 260 may interface or communicate with other components in the system 100. Other components in the motion monitoring system 100 may be connected or communicated through the input/output module 260.

It should be noted that the above descriptions of the system 100 and the modules thereof are merely for the convenience of descriptions, and cannot limit one or more embodiments of the present disclosure to the scope of the illustrated embodiments. It can be understood that for those skilled in the art, after understanding the principle of the system, it is possible to arbitrarily combine the various modules, or form a subsystem to connect with other modules, or omit one or more modules. For example, the acquisition module 210 and the processing module 220 may be integrated into one module which may have the functions of obtaining and processing the user action signal. As another example, the processing module 220 may not be provided in the wearable apparatus 130 but integrated in the processing device 110. Such modifications are within the protection scope of one or more embodiments of the present disclosure.

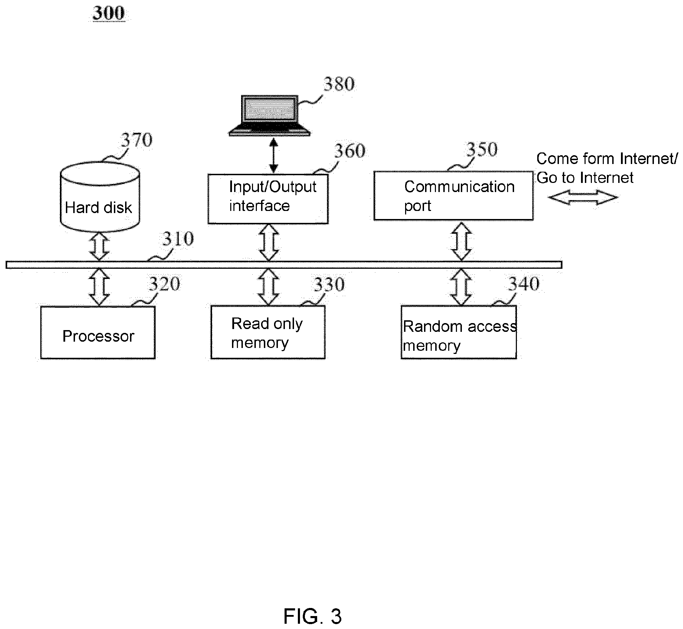

FIG. 3 illustrates a schematic diagram of exemplary hardware and/or software components of a computing device 300 according to some embodiments of the present disclosure. In some embodiments, the processing device 110 and/or the mobile terminal device 140 may be implemented on the computing device 300. In some embodiments, the action data presentation system 160 may be implemented on the computing device 300. As shown in FIG. 3, the computing device 300 may include an internal communication bus 310, a processor 320, a read-only memory 330, a random-access memory 340, a communication port 350, an input/output interface 360, a hard disk 370, and a user interface 380.

The internal communication bus 310 may enable data communication among the various components of the computing device 300. For example, the processor 320 may send data to memory or other hardware components such as the input/output interface 360 through the internal communication bus 310.

The processor 320 may perform a computing instruction (a program code) and perform the functions of the motion monitoring system 100 described herein. The computing instruction may include a program, an object, a component, a data structure, a procedure, a module, and a function (the function refer to the specific function described in the disclosure). For example, the processor 320 may process an action signal (e.g., an EMG signal, a posture signal) obtained from the wearable device 130 or/and the mobile terminal device 140 of the motion monitoring system 100 during the user's motion, and monitor the action of the user according to the action signal during the user's motion. For illustration only, the computing device 300 in FIG. 3 only depicts one processor, but it should be noted that the computing device 300 in the present disclosure may also include a plurality of processors.

The memory (e.g., a read-only memory (ROM) 330, a random-access memory (RAM) 340, a hard disk 370, etc.) of the computing device 300 may store data/information obtained from any other component of the motion monitoring system 100. In some embodiments, the memory of computing device 300 may be located in wearable apparatus 130 as well as in processing device 110.

The input/output interface 360 may be configured to input or output a signal, data, or information. In some embodiments, the input/output interface 360 may allow a user to interact with the motion monitoring system 100.

The hard disk 370 may be configured to store information and data generated by or received from the processing device 110. For example, the hard disk 370 may store user confirmation information of the user. In some embodiments, the hard disk 370 may be provided in the processing device 110 or in the wearable apparatus 130. The user interface 380 may enable interaction and exchange of information between the computing device 300 and the user. In some embodiments, the user interface 380 may be configured to present motion recordings generated by the motion monitoring system 100 to the user. In some embodiments, the user interface 380 may include a physical display, such as a display with a speaker, an LCD display, an LED display, an OLED display, an electronic ink display (E-Ink), or the like.

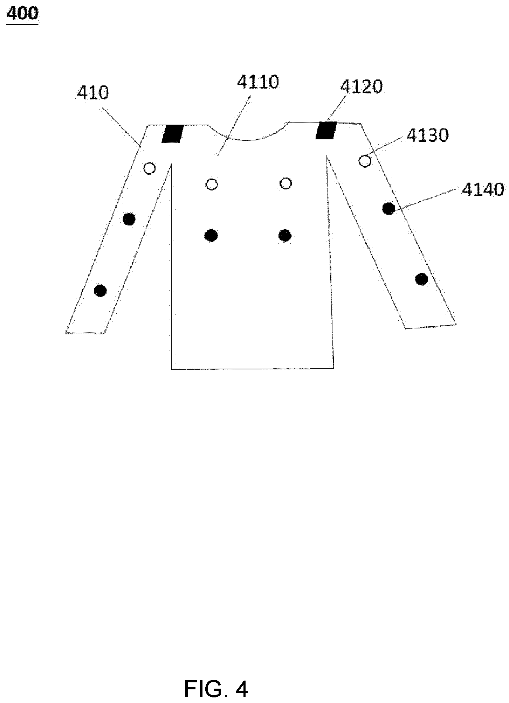

For example, the wearable apparatus 130 in the system 100 may adopt any structure. For example, the wearable apparatus 130 may adopt a structure of the wearable apparatus 400 shown in FIG. 4. In order to describe the wearable apparatus 130, the wearable apparatus 400 in FIG. 4 may take the clothes above as an example. As shown in FIG. 4, the wearable apparatus 400 may include an upper garment 410. The upper garment 410 may include an upper garment base 4110, one or more upper garment processing modules 4120, one or more upper garment feedback modules 4130, one or more upper garment acquisition modules 4140, or the like. The upper garment base 4110 may refer to a clothing worn on the upper body of the human body. In some embodiments, the upper garment base 4110 may include a short-sleeved T-shirt, a long-sleeved T-shirt, a shirt, a jacket, or the like. The one or more upper garment processing modules 4120 and the one or more upper garment acquisition modules 4140 may be located on the upper garment base 4110 in areas that fit with different portions of the human body. The one or more upper garment feedback modules 4130 may be located at any position on the upper garment base 4110, and the one or more upper garment feedback modules 4130 may be configured to feed back motion state information of the user's upper body. Exemplary feedback techniques may include, but are not limited to, a voice prompt, a text prompt, a pressure prompt, an electrical stimulation, or the like. In some embodiments, the one or more the upper garment acquisition modules 4140 may include, but are not limited to one or more of a posture sensor, an ECG sensor, an EMG sensor, a temperature sensor, a humidity sensor, an acid-base sensor, a sound wave transducer, or the like. The sensors in the upper garment acquisition module 4140 may be placed at different positions on the user's body according to different signals to be measured. For example, when the posture sensor is configured to obtain a posture signal during the user's motion, the posture sensor may be placed in the positions of the upper garment base 4110 corresponding to the torso, arms, and joints. As another example, when the EMG sensor is configured to obtain an EMG signal during the user's motion, the EMG sensor may be located near the muscles to be measured by the user. In some embodiments, the posture sensor may include, but is not limited to, an acceleration triaxial sensor, an angular velocity triaxial sensor, a magnetic force sensor, etc., or any combination thereof. For example, the posture sensor may include an acceleration triaxial sensor and an angular velocity triaxial sensor. In some embodiments, the posture sensor may also include a strain gauge sensor. The strain gauge sensor may refer to a sensor that may be based on the strain generated by the force and deformation of an object to be measured. In some embodiments, the strain gauge sensor may include, but is not limited to, one or more of a strain gauge load cell, a strain gauge pressure sensor, a strain gauge torque sensor, a strain gauge displacement sensor, a strain gauge acceleration sensor, or the like. For example, the strain gauge sensor may be set at the user's joint position, and by measuring the resistance of the strain gauge sensor that changes with the stretched length, a bending angle and a bending direction of the user's joint may be obtained. It should be noted that in addition to the above-mentioned upper garment base 4110, the upper processing module 4120, the upper garment feedback module 4130, and the upper garment acquisition module 4140, the upper garment 410 may also include other modules, such as a power supply module, a communication module, an input/output module, etc. The upper garment processing module 4120 may be similar to the processing module 220 in FIG. 2, and the upper garment acquisition module 4140 may be similar to the acquisition module 210 in FIG. 2. For more descriptions of the various modules of the upper garment 410, please refer to FIG. 2 and the related descriptions thereof in the present disclosure, which are not repeated here.

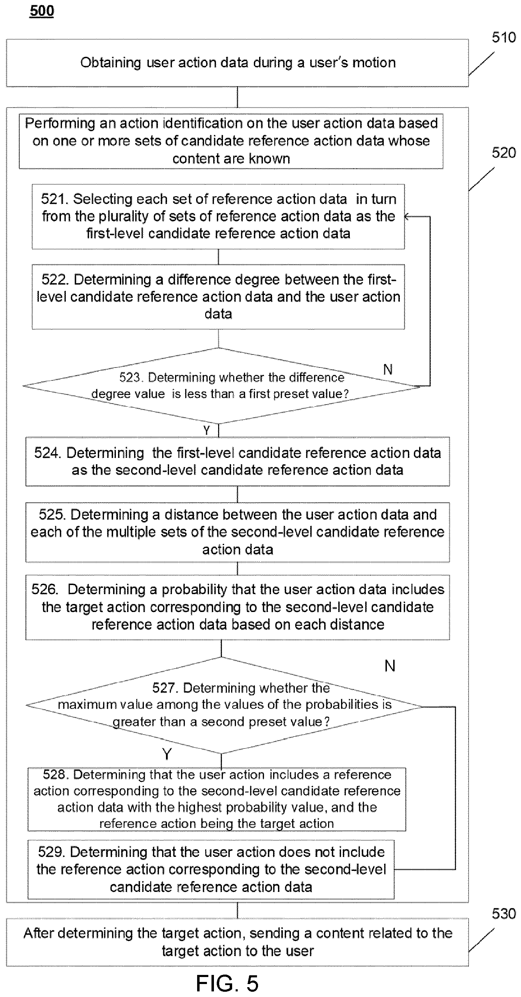

FIG. 5 is a flowchart illustrating an exemplary process for determining a target action according to some embodiments of the present disclosure. In some embodiments, the process 500 may be implemented by the system 100. For example, the memory in the processing device 110 and/or the mobile device 140 may store one or more sets of action analysis and identification instructions. The set of instructions may include a plurality of instructions. The processor of the processing device 110 and/or the mobile device 140 may read and execute the plurality of instructions in the set of instructions at runtime, and execute the process 500 under the guidance of the plurality of instructions. The process 500 may be completed in real-time, or each operation may be completed in different time periods. The process 500 may include the following operations.

In operation 510, user action data during a user's motion may be obtained. The user action data may correspond to an unknown user action.

The processing device 110 and/or the mobile device 140 may measure the above-mentioned action data from a plurality of measurement positions on the user, for example, may obtain raw data during the user's motion. Specifically, the processing device 110 and/or the mobile device 140 may be communicatively connected with the wearable apparatus 130 directly or through the network 120. The wearable apparatus 130 may have a plurality of sensors. When the user wears the wearable apparatus 130, the plurality of sensors may be attached to a plurality of positions on the user's body. Therefore, the processing device 110 and/or the mobile device 140 may obtain measurement results of the plurality of sensors attached to the user at the plurality of measurement positions through the corresponding acquisition modules to obtain the action data of the user.

The user action data may refer to action data generated based on human body parameter information during the user's motion. In some embodiments, the human body parameter information may include, but is not limited to, one or more of an EMG signal, a posture signal, an electrocardiographic signal, a temperature signal, a humidity signal, a blood oxygen concentration, a respiratory rate, or the like.

Since a user action is a coordination result of a plurality of muscles and joints, correspondingly, the user action data may also include data collected by multiple sensors at M positions on the user's body, wherein M is an integer greater than 1. In some embodiments, the data collected by each individual sensor may be considered as a piece of action sub-data. For example, in some embodiments, the plurality of sensors in the wearable apparatus 130 may obtain signals of a plurality of body parts during the user's motion. The combination of the gesture signals of the plurality of body parts may reflect the relative motion situation between different parts of the human body. For example, an EMG sensor in the wearable apparatus 130 may collect an EMG signal during the user's motion, a posture sensor in the wearable apparatus 130 may collect a posture signal during the user's motion, and an angle sensor and an angular velocity sensor in the wearable device 130 may collect an angle and an angular velocity of each joint during the user's motion. The signal of each sensor of the above-mentioned sensors is recorded as a piece of action sub-data. All the action sub-data may be combined to form the action data.

For example, when a person performs an arm curling action, the corresponding action data may include angle data and angular velocity data of the upper arm, angle data, and angular velocity data between the upper arm and the forearm, EMG data of biceps brachii muscle, EMG data of deltoid muscle, EMG data of trapezius muscle, and data of back muscle group, etc., measured by the wearable apparatus 130. As another example, when the user performs seated chest clamping, the EMG sensor in the wearable apparatus 130 corresponding to the position of the human body's pectoralis muscle, latissimus dorsi, etc., may collect the EMG signal of the user's corresponding muscle position. As further another example, when the user performs a squat, the EMG sensor in the wearable apparatus 130 corresponding to the position of the human gluteus maximus, quadricep, etc., may collect the EMG signal of the user's corresponding muscle position, and the nearby angle sensor and the angular velocity sensor may collect the angle and the angular velocity between the thigh and the calf. The data collected by each single sensor may be regarded as a piece of action sub-data. Therefore, the user action data of the arm curling action may also include a plurality of user action sub-data, respectively corresponding to the data collected at the plurality of positions of the user's body when the user performs the arm curling action.

When the user wears the wearable apparatus 130, the wearable apparatus 130 may start to measure the user's motion at any time, and the user may start to exercise or start to rest at any time. Thus, the processing device 110 and/or the mobile device 140 do not know whether the wearable apparatus 130 collects a segment of user's random action data or the aforementioned fitness action, nor does the processing device 110 and/or the mobile device 140 know when did the fitness action start in the collected data. Therefore, what the wearable apparatus 130 collects is action data whose content is unknown. That is to say, the system 100 is in an unknown state about what kind (that is, whether the user action includes several known target actions, and a start time of each target action) of motion the user is doing and when the user starts exercising. Therefore, the action data also has no identifier indicating the content of the action. Merely for the convenience of description, the present disclosure may take whether the action data includes an action identifier as an example to describe whether the content of the action data is known.

In operation 520, whether the user action includes a reference action (i.e., a target action) corresponding to one or more sets of candidate reference action data may be identified based on the one or more sets of candidate reference action data whose content is known by performing action identification on the user action data.

The target action may refer to a specific action in the actual actions performed by the user, such as biceps curling, bench press, squat, kick, push-up, deadlift, abdominal crunches, etc. The reference action may refer to a standard action with an action content marked on the specific action performed by a reference person (such as a coach, etc.). In some embodiments, the target action identification may be performed immediately or within a preset time after the user action data is obtained. The preset time may be a very short time, for example, within one hour or 0.1, 0.5 seconds. The target action identification may also be performed in real time with the acquisition of the user action data. For example, when the user is performing an action, the processor of the processing device 110 and/or the mobile device 140 may obtain the user action data while simultaneously stretching or compressing the user action data to a different scale and then comparing it to the reference action data, thereby simultaneously performing the action identification on the user action data. The method of comparison described above may be described in other parts of the present disclosure.

The reference action data may include a plurality of sets of reference action data measured by a plurality of sensors. When collecting the reference action data, similar to the collection of the user action data, the plurality of sensors may also be attached to the M measurement positions of the reference person, for example, the reference person may wear the wearable apparatus 130. Then the reference person may perform the specific action, and the data acquisition device (e.g., the processing device 110 and/or the mobile device 140, or other devices) may receive the corresponding action data through the corresponding acquisition module in the wearable apparatus 130. Therefore, the candidate reference action data may be action data of a reference action whose content is known and measured from the M measurement positions on the reference person, such as reference action data marked with an action content. For example, the reference action data of the upper arm curling may include data collected from the same plurality of positions on the reference person. The reference person may be a person who is used as a model to collect the reference action data, such as a fitness coach. For the data collected by each sensor, the reference action data of the arm curling may include M pieces of reference action sub-data each of which corresponds to the data collected at one of the M positions of the body when the reference person performs the arm curling action. In some embodiments, the M measurement positions on the reference person may have the one-to-one correspondence relationship with the M measurement positions on the user when the user action data is collected. Correspondingly, the M pieces of user action sub-data of the user action data may have the one-to-one correspondence relationship with the M pieces of reference action sub-data.

The reference action data may also be generated in other ways. For example, action data of a virtual person may be obtained through computer modeling, and the reference action data may be obtained by artificially fitting the virtual person in the video through artificial intelligence techniques (for example, artificially fitting Mr. Olympia's action demonstration video). As long as certain action data may standardly represent a certain action, and an action content of the certain action data is known, the certain action data may be used as the reference action data in the present disclosure. As mentioned above, merely for the convenience of description, the present disclosure may take the action data marked with the content as an example to describe the action data with known content.

In operation 520, the processing device 110 and/or the mobile device 140 may access a reference action database. The reference action database may include the plurality of sets of reference action data. The plurality of sets of reference action data may correspond to a plurality of reference actions. For example, each set of the reference action data may correspond to one reference action, or each set of the reference action data may correspond to a plurality of reference actions, or each reference action may correspond to the plurality of sets of reference action data.

When performing target action identification on the user action data, the processing device 110 and/or the mobile device 140 may identify the user action data by sequentially comparing the user action data with each of the plurality of sets of reference action data in the reference action database through a two-level screening operation. The two-level screening operation may screen the plurality of sets of reference action data through two different screening operations, and finally determine which reference action the user's action includes. For example, the two-level screening operation may include a combination of a difference degree-based screening operation and a probability-based screening operation.

Specifically, in the first level screening, the processing device 110 and/or the mobile device 140 may select a set of candidate reference action data as first-level candidate reference action data, and then use the selected candidate reference action data to determine a difference degree of the action with the user action data to determine whether a difference in data values between the user action data and the first-level candidate reference action data is sufficiently small. If the difference degree between the user action data and a certain first-level candidate reference action data is less than a preset value, the first-level candidate reference action data may be promoted to a second-level candidate reference action data. Each second-level candidate reference action data corresponds to one or more promoted reference actions, that is, the second-level reference action.

The second level screening may be a probabilistic screening. In the second level screening, the processing device 110 and/or the mobile device 140 may determine the probability that the user action includes the promoted reference action (second-level reference action), and then determine whether the user action includes the second-level reference action. Whether the user action includes the target action corresponding to target reference action data may be determined based on the result of the second level screening. Specific operations are described as follows.

In operation 521, each set of reference action data may be selected in turn from the plurality of sets of reference action data as the first-level candidate reference action data.

In operation 522, a difference degree between the first-level candidate reference action data and the user action data may be determined.

In operation 523, whether a value of the difference degree is less than a first preset value may be judged. If the difference degree value is greater than or equal to the first preset value, the overall difference between the user action and the first-level candidate reference action data may be considered relatively large. Then operation 521 may be performed, that is, the next set of reference data may be selected from the plurality of sets of reference action data in the reference action database as the first-level candidate reference action data and the next set of reference data may be compared with the user action data again at the data value level. If the difference degree value of the next set of reference data is less than the first preset value, the overall difference between the user action and the first-level candidate reference action data may be considered small. Then operation 524 may be performed, that is, the first-level candidate reference action data may be determined as the second-level candidate reference action data, and then the next-level target action data identification may be performed.

In operation 525, a distance between the user action data and each of the multiple sets of the second-level candidate reference action data may be determined.

In operation 526, a probability that the user action data includes the target action corresponding to the second-level candidate reference action data may be determined based on each distance.

In operation 527, whether the maximum value among the values of the probabilities is greater than a second preset value is judged. If the maximum value is not greater than the second preset value, operation 529 may be performed, that is, it is determined that the user action does not include the reference action corresponding to the second-level candidate reference action data. If the maximum value is greater than the second preset value, operation 528 may be performed, that is, it is determined that the user action includes the reference action corresponding to the second-level candidate reference action data with the highest probability value, and the reference action is the target action.

The basis for selecting the first-level candidate reference action data may be random and in sequence, or may be selected according to a certain rule, which is not limited in the present disclosure. For example, all reference action data may be numbered in the reference action database in advance, and then the processing device 110 and/or the mobile device 140 may select the reference action data item by item as the first-level candidate reference action data according to the number.

When comparing the first-level candidate reference action data with the user action data, a sliding window comparison manner may be adopted. For example, the processing device 110 and/or the mobile device 140 may slide a sliding window over the user action data along the time axis and select a segment of the user action data within the sliding window. Since the M pieces of user action sub-data in the user action data are collected in parallel, the sliding window may act on each piece of user action sub-data at the same time, and slide over each piece of user action sub-data in parallel. The sliding window may correspond to a preset time interval (such as 0.1 seconds, 0.5 seconds, 1 second, etc.). Therefore, for the M pieces of user action sub-data, the sliding window may include M data segments of the user action data collected in the preset time interval. The processing device 110 and/or the mobile device 140 may respectively compare the M user action data segments with some or all of the data of the M pieces of reference action sub-data corresponding to the first-level candidate reference action data to obtain one or more comparison sub-results and then determine a comprehensive difference degree by weighting and summing the one or more comparison sub-results. The comprehensive difference degree indicates the difference between the user action data and the reference action data. The smaller the value of the comprehensive difference degree is, the smaller the difference is, which indicates that the closer the user action data segment is to the reference action data, the closer the user action corresponding to the user action data segment is to the reference action, and the processing device 110 and/or the mobile device 140 may determine that the user action data includes the reference action. For example, when a user performs biceps curling during the user's motion, the user action data may include a corresponding user action data segment. When the processing device 110 and/or the mobile device 140 compares the user action data segment corresponding to the biceps curling with the reference action data corresponding to the biceps curling, the comprehensive difference degree value may be very small. On the other hand, the smaller the value of the comprehensive difference degree may indicate that the closer the position of the user action data segment in the user action data is to the position of the target action in the user action data, that is, the user action corresponding to the user action data segment is closer in time to the moment when the user performs the target action.

Specifically, the processing device 110 and/or the mobile device 140 may use a sliding window with a preset width to slide over the user action data along the time axis with a preset step size, and select a user action data segment within the sliding window each time. For example, the processing device 110 and/or the mobile device 140 may sequentially select a segment of continuous data with a preset data length on the user action data with the preset step size. Considering that the speed of the user performing the target action may be different from the speed of the standard action performed by the reference person, the sliding window length may be negatively correlated with the use action speed to offset the difference. That is, when the user action speed is faster, the taken sliding window length is shorter, and when the user action speed is slower, the taken sliding window length is longer.

The preset step size may be a constant value. Since the value of the comprehensive difference degree also denotes a temporal distance between the user action corresponding to the current user action data segment and the target action, the preset step size may also be adjusted based on the value of the comprehensive difference degree. For example, in order to increase the efficiency of identifying the target action, the preset step size may be positively correlated with the magnitude of the value of the comprehensive difference degree at the previous moment. The positive correlation may indicate that the preset set size is proportional to the value of the comprehensive difference degree at the previous moment, or may select the step size of the current moment in a certain step manner based on the value of the comprehensive difference degree at the previous moment, or may be that the step size of the current moment is greater than the step size of the previous moment by a constant, etc., which is not limited here. The preset step size may also be positively correlated with the variation trend of the value of the comprehensive difference degree. For example, if the difference between the comprehensive difference degree value at the current moment and the comprehensive difference degree value at the previous moment is greater than 0, i.e., the variation trend of the comprehensive difference degree value is increasing, which means that the user action corresponding to the current user action data segment is getting farther and farther away from the target action in time. At this time, the processing device 110 and/or the mobile device 140 may increase the step size. If the difference between the comprehensive difference degree value at the current moment and the comprehensive difference degree value at the previous moment is less than 0, which means that the user action corresponding to the current user action data segment is getting closer and closer to the target action in time. At this time, the processing device 110 and/or the mobile device 140 may reduce the step size. If the difference between the comprehensive difference degree value at the current moment and the comprehensive difference degree value at the previous moment is equal to 0, the step size may be kept unchanged.

Since the width of the sliding window is preset, the length of the data segment intercepted from the user action data may also be preset. Therefore, the user action data segment may correspond to the entire first-level candidate reference action data. The user action data segment may also correspond to a portion of the first-level candidate reference action data. In some embodiments, the reference action data may include one or more segments of action identification data. The action identification data may be action data (e.g., angular velocity data, velocity data, etc.) corresponding to at least a portion of the characteristic action of the reference action, which is essentially configured to represent the characteristic action. The characteristic action may be unique to the reference action. The reference action may be determined through the portion of the characteristic action, or the entire data may be determined as the reference action data through the action identification data, so that when a data segment similar to the action identification data appears in the user action data, the user action may be recognized as including the corresponding target action. Meanwhile, the action identification data may only exist on a portion of the reference action sub-data of the reference action data, or the action identification data may exist in each reference action sub-data. The action identification data existing on the reference action sub-data may be referred to as action identification sub-data.

For the sliding window at the current moment, the processing device 110 and/or the mobile device 140 may compare the M user action data segments with the corresponding M pieces of action identification sub-data respectively to obtain the corresponding M difference degrees. The M difference degrees may be weighted and summed to obtain the comprehensive difference degree. If the comprehensive difference degree is less than the first preset value, the first-level candidate reference action data having passed the first level screening may be determined, and the first-level candidate reference action data may be selected as the second-level candidate reference action data. If the value of the comprehensive difference degree is greater than the first preset value, the sliding window may slide to the next user action data segment with the preset step size, and then the comparison may be repeated until the comprehensive difference degree value is less than the first preset value or the sliding window slides to the end of the user action data.

Specifically, when comparing a certain segment of action identification sub-data of a certain piece of first-level candidate reference action data with its corresponding user action sub-data, the following operations may be performed.

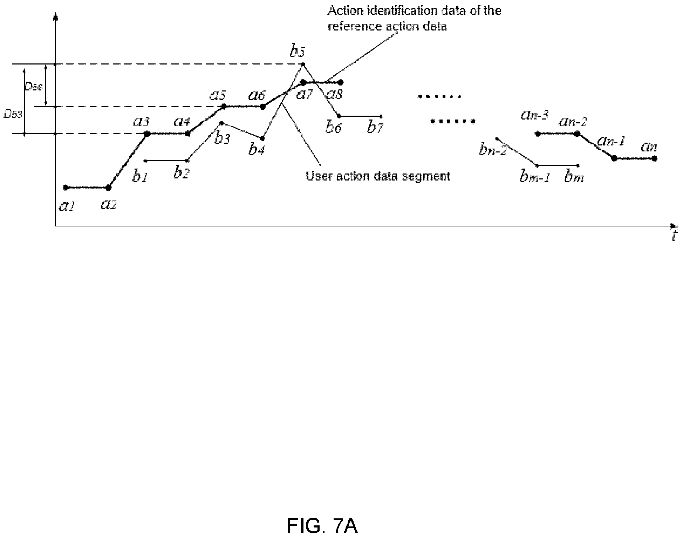

For a certain segment of action identification sub-data in the first-level candidate reference action data, the data is two-dimensional. For example, the first-level candidate reference action data of the arm curling may include action identification sub-data at different time points for the bending angle of the forearm relative to the upper arm. The action identification sub-data may include a plurality of angle values and a plurality of corresponding time points, so the action identification sub-data is two-dimensional data. For such single parameter action identification sub-data, when a segment of action identification sub-data is included in one piece of the first-level candidate reference action data, the specific process for obtaining the comprehensive difference degree is as follows. FIG. 7A shows an exemplary segment of action identification data in reference action data and a curve of a segment of the user action sub-data collected by the sliding window in the user action data on the time axis according to some embodiments of the present disclosure. The action identification sub-data in the reference action data may include a plurality of pieces of data $\{a_j\}=\{a_1, a_2, a_3, ..., a_n\}$, wherein $n$ is an integer greater than 1, each piece of the data corresponds to a timestamp, and as $j$ increases, the time point corresponding to the timestamp of each data $a_j$ increases sequentially. That is, the data points in the vector $\{a_j\}$ may be arranged in chronological order. The user action sub-data segment may include a plurality of pieces of data $\{b_i\}=\{b_1, b_2, a_3, ..., b_m\}$, wherein $m$ is an integer greater than 1, each piece of the data corresponds to a timestamp, and as $i$ increases, the time point corresponding to the timestamp of each data $bi$ increases sequentially. That is, the data points in the vector $\{b_i\}$ a may be arranged in chronological order. Generally speaking, the time length corresponding to the sliding window is less than the time length corresponding to the action identification sub-data, and the amount of data corresponding to the sliding window is less than the amount of data corresponding to the automatic identification data, that is, $m<n$.

Assuming that sampling frequencies and action speeds of the reference action data and the user action data are the same, for the same time interval, the quantity of data points of the reference action data and the quantity of data points of the user action data may be the same. Thus, the user action sub-data segment $\{b_i\}$ may correspond to the data of the same length in the action identification sub-data $\{a_j\}$. That is, each data point in $\{b_i\}$ may correspond to a data point in $\{a_j\}$. When determining the difference degree between $\{b_i\}$ and $\{a_j\}$, $\{b_i\}$ only needs to be drawn along the time axis t, a distance of one data point can be slid each time, and once difference degree determination between the data point and the corresponding data point in $\{a_j\}$ may be determined every time. However, considering that the sampling frequencies and/or the action speeds of the reference action data and the user action data are inconsistent, there is no one-to-one correspondence between the data points of the user action and the data points of the reference action. At this time, an action-time relationship of $\{b_i\}$ needs to be adjusted according to various time scales to make the adjusted action-time relationship consistent with the action-time relationship of the reference action data. For example, if the sampling frequencies of the reference action data and the user action data are the same but the action speeds are inconsistent, the time taken by the user to perform an action is different from the time taken by the corresponding reference action. For example, if the hardware sampling frequency is 100 data points per second, a speed of a joint in the reference action changes from 0° to 90° in 1 second, and a speed of the user action may only change from 0° to 45° in 1 second, for 100 data points, the reference action data corresponds to an angle change of 90° and the user action data corresponds to an angle change of 45°. Thus, the time span of $\{b_i\}$ needs to be adjusted according to various time scales, i.e., stretching or compressing $\{bi\}$ according to different scales, and then discriminating the difference degree between the processed data point and the corresponding data point in $\{a_j\}$ once for each scale, until all the difference degrees corresponding to all scales are determined. The specific operations may be described as follows.

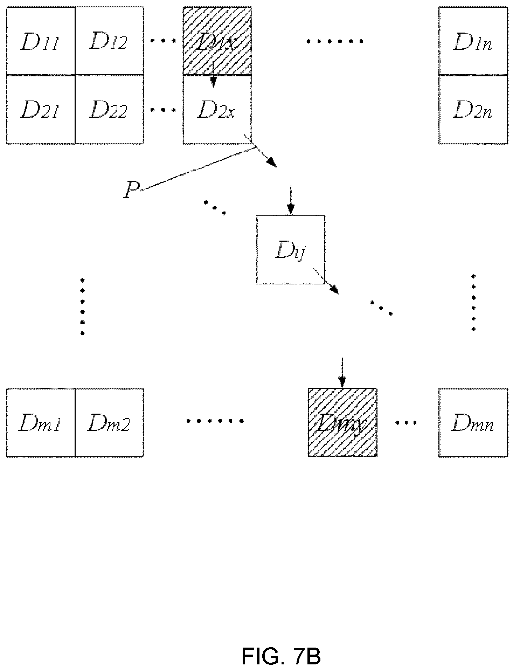

Firstly, the processing device 110 and/or the mobile device 140 may calculate a distance $D_{ji}$ between any point $b_i$ in the user action sub-data and any point $a_j$ in the action identification sub-data by placing the user action sub-data segment $\{b_i\}$ and the action identification sub-data $\{a_j\}$ on the same time axis, and determine the m×n distance matrix $\mathbf{D}_{m \times n}$ as shown in FIG. 7B. Each element in the distance matrix denotes a distance from the i-th (i≤m) point of the user action sub-data segment to the j-th (i≤n) point of the action identification sub-data in the first-level candidate reference action data. Taking a fitness action such as biceps curling as an example of the user action, the user action sub-data $\{bi\}$ may include angles between the upper arm and the forearm, and the corresponding action identification sub-data $\{aj\}$ in the reference data may also include angles between the upper arm and the forearm. Thus, the distance $D_{ji}$ may indicate a difference $D_{ji}=|a_j - b_i|$ between the angle between the upper arm and the forearm of the user and the angle represented by the action identification sub-data. The distance $D_{56}$ between $a_5$ and $b_6$ and the distance $D_{53}$ between $a_5$ and $b_3$ are shown in FIG. 7A.

Of course, the distance may also be a distance defined in other ways. For example, a distance between any point $b_i$ in the user action sub-data and any point $a_j$ in the action identification sub-data may be a Euclidean distance, a Manhattan distance, a P-parametric distance, a cosine distance, a Chebyshev distance, a Marxian distance, an edit distance, a Jaccard distance, or any other correlation distance. In this way, the distance matrix $\mathbf{D}_{m\times n}$ includes point-to-point distances between all points in the user action sub-data segment $\{b_i\}$ on all scales and all points in the action identification sub-data $\{a_j\}$.

Secondly, the processing device 110 and/or the mobile device 140 may determine the shortest distance path $P_{min}$ in the distance matrix $\mathbf{D}_{m\times n}$, i.e., the smallest regularization cost. The distance path may be expressed by the following vector, $P=\{p_k\}=\{p_1, p_2, ......, p_l\}$, which is a sequence composed of some elements of the distance matrix $\mathbf{D}_{m\times n}$, wherein $l$ denotes a count of elements in the distance path P. The distance path P may include a plurality of numbers, each of which is an element (i.e., a distance) in $\mathbf{D}_{m\times n}$. Any two adjacent numbers are two adjacent elements of the distance matrix $\mathbf{D}_{m\times n}$, and a position of the next number of the sequence in the distance matrix $\mathbf{D}_{m\times n}$ is to the right, below, or below the right of a corresponding position of the previous number of the sequence in the distance matrix $\mathbf{D}_{m\times n}$. Since the time corresponding to the user action sub-data segment in the sliding window is shorter than the time corresponding to the action identification sub-data, two ends of the shortest distance path P may correspond to the first data point $b_1$ and the last data point $b_m$ of the $\{b_i\}$, that is, the first number in the sequence is $p_1=D_{1x}$, and the last number is $p_l=D_{my}$, wherein $x<n$, $y<n$, x and y denote the positions of the corresponding data points in $\{a_j\}$, respectively. The shortest distance path $P_{min}$ is the one with the smallest sum of all elements among all the paths satisfying the above conditions, that is, $P_{min} =$

$$P|_{\min(\sum_{k=1}^{l} p_k)} = \{p_1, p_2, p_3, ... p_l\}|_{\min(\sum_{k=1}^{l} p_k)}.$$

Considering that the sliding window may be set with different time lengths and different data sampling frequencies, the quantity of data points in the user action data segment in the time window may be different. This may cause the value of the shortest distance path P to be different, depending on the data sampling frequency and the length of the time window of the sensors. Taking these factors into consideration, the difference degree $f$ may be defined as

$$f = WP_{min}^T$$, wherein $WP_{min}^T = \sum_{i=1}^{l} w_i p_i$ may be the weighted average of the elements of the shortest distance path $P_{min}$, $W = \{w_1, w_2, ... w_l\}$ may be a weight vector consisting of $m$ elements with 1 row and $l$ columns, and

$$WP_{min}^T$$ is a scalar. For example, the difference degree may be defined as an average distance, that is, all elements in $W$ are $1/l$.

Through the above calculation, the processing device 110 and/or the mobile device 140 may complete the operations of determining the difference degree between each of the plurality of scaled data segments and the action identification sub-data, respectively, and determining the smallest difference degree among difference degrees between the data segment and the action identification sub-data.

In some embodiments, in order to reduce the amount of computation and determine the adjustment scale of the user action sub-data, before determining the difference degree of the user action sub-data segment within the sliding window, the processing device 110 and/or the mobile device 140 may determine a target comparison data interval from the action identification sub-data, and only compare the target comparison data interval with the user action sub-data segment to obtain a difference degree. The target comparison data interval may include a plurality of action identification sub-data points. The target comparison data interval may be determined based on the following relationship between the user action data and the action identification sub-data selected by the sliding window.

1) The beginning and end of the user action data segment selected by the sliding window exactly correspond to the beginning and end of the action identification sub-data, that is, the user action data segment is exactly the complete distribution of the action identification sub-data. In this case, the processing device 110 and/or the mobile device 140 may first determine that the first data point and the last data point of the user action data segment selected by the sliding window correspond to the first data point and the last data point of the corresponding action identification sub-data, respectively. The target comparison data interval covers the entire action identification sub-data. The constraint of the distance path P may include that: each number of the sequence is an element of $\mathbf{D}_{m\times n}$, any two adjacent numbers are two adjacent elements in the distance matrix $\mathbf{D}_{m\times n}$, and the position of the next number of the sequence in the distance matrix $\mathbf{D}_{m\times n}$ is to the right, below, or below the right of the corresponding position of the previous number in the distance matrix $\mathbf{D}_{m\times n}$. The two ends of the shortest distance path P may correspond to $p_1=D_{11}$ and $p_l=D_{mn}$. That is, the shortest distance path P of the distance matrix is the shortest distance path from the upper left corner to the lower right corner of the distance matrix.

2) The start point of the user action sub-data selected by the sliding window corresponds to the start point of the action identification sub-data, and the end point of the user action sub-data selected by the sliding window corresponds to a certain data point in the action identification sub-data. That is, the user action sub-data corresponds to a segment in the action identification sub-data after scaling, and this segment is located at the start position of the action identification sub-data. In this case, the processing device 110 and/or the mobile device 140 may first determine that the first data point of the user action data segment selected by the sliding window corresponds to the first data point of the corresponding action identification sub-data. Then, the target comparison data interval may cover the entire action identification sub-data. The constraint of the distance path P may include that: each number of the sequence is an element in $\mathbf{D}_{m \times n}$; any two adjacent numbers are two adjacent elements of the distance matrix $\mathbf{D}_{m \times n}$, and the position of the next number of the sequence in the distance matrix $\mathbf{D}_{m \times n}$ is to the right, below, or below the right of the corresponding position of the previous number in the distance matrix $\mathbf{D}_{m \times n}$. Both ends of the shortest distance path P may correspond to $p_1=D_{11}$ and $p_l=D_{my}$. That is, the shortest distance path P of the distance matrix is a distance from the upper left corner of the distance matrix to a certain point of the last row along the lower right direction.

3) The start point of the user action sub-data selected by the sliding window corresponds to a certain data of the action identification sub-data, and the end point of the user action sub-data selected by the sliding window corresponds to the end point of the action identification sub-data. That is, after scaling, the user action sub-data may be a segment of data at the end position of the action identification sub-data. In this case, the processing device 110 and/or the mobile device 140 may first determine that the last data point of the user action data segment selected by the sliding window corresponds to the last data point of the corresponding action identification sub-data. Then, the target comparison data interval may cover the entire action identification sub-data. The constraint of the distance path P may include that: each number of the sequence is an element in $\mathbf{D}_{m \times n}$, any two adjacent numbers are two adjacent elements of the distance matrix $\mathbf{D}_{m \times n}$, and the position of the next number of the sequence in the distance matrix $\mathbf{D}_{m \times n}$ is to the right, below, or below the right of the corresponding position of the previous number in the distance matrix $\mathbf{D}_{m \times n}$. Both ends of the shortest distance path P may correspond to $p_1=D_{1x}$ and $p_l=D_{mn}$. That is, the shortest distance path P of the distance matrix is the shortest distance path from a certain point in the first row of the distance matrix to the lower right corner.

4) The start point and the end data point of the user action sub-data selected by the sliding window correspond to the two intermediate data points of the action identification sub-data, respectively, rather than the first data point and the last data point thereof. That is, the start point of the user action sub-data selected by the sliding window may not be the beginning of the action identification sub-data, and the end point of the user action sub-data selected by the sliding window may also be not the end of the action identification sub-data. After scaling, the user action sub-data may be a segment of data of the action identification sub-data, and this segment may be located at a certain position in the middle of the action identification sub-data. The "intermediate data" of a segment of data may refer to data at any position except the start point and the end point of the data. In this case, the processing device 110 and/or the mobile device 140 may first determine that the first data point and last data point of the user action sub-data segment selected by the sliding window may not be the first data point and the last data point of the corresponding action identification sub-data. Then the target comparison data interval may cover the entire action identification sub-data except for the first data point and the last data point. The constraint of the distance path P may include that: each number of the sequence is an element in $\mathbf{D}_{m \times n}$, any two adjacent numbers are two adjacent elements of the distance matrix $\mathbf{D}_{m \times n}$, and the position of the next number of the sequence in the distance matrix $\mathbf{D}_{m \times n}$ is to the right, below, or below the right of the corresponding position of the previous number in the distance matrix $\mathbf{D}_{m \times n}$. The two ends of the shortest distance path P may correspond to $p_l=D_{1x}$ and $p_l=D_{my}$, wherein, $x \in (1, y]$, $y \in [1, n)$. That is, the shortest distance path P of the distance matrix starts from a certain point in the middle of the first row of the distance matrix and extends to the lower right and ends at a certain point in the last row.

In some embodiments, the action identification sub-data may correspond to a start action or an end action of a reference action. At this time, when determining whether a certain point in the user action sub-data is the start point or the end point of the action, it may be determined by the change of the angular velocities before the point and after the point. For example, when a user action data point shows that the angular velocity of the corresponding user action is 0, and the angular velocity of a point after the user action data point is not 0, it may be proved that the user starts a certain fitness action from the user action data point, so it may be determined that the user action data point is the start point of the user action. As another example, when the angular velocity of a user's action point is 0, and the angular velocity of the previous point is not 0, it may be proved that the user stopped doing the action at this point, so it may be determined that the user's action point is the end point of the action.

It should be understood that performing target action identification on the user action data may include one of the following two cases. 1) The action identification sub-data may be compared with the user action sub-data segment by segment to obtain a comprehensive difference degree value. When the value of the comprehensive difference degree is less than the first preset value, the processing device 110 and/or the mobile device 140 may determine that the first-level candidate reference action data is the second-level candidate reference action data. 2) The action identification data may be compared with the user action data segment by segment to obtain a comprehensive difference degree value. When the value of the comprehensive difference degree is greater than the first preset value, the processing device 110 and/or the mobile device 140 may slide the sliding window to the next data segment at the preset step size, and then repeat the comparison. The first preset value may be a criterion for determining whether the distance between the user action data and the action corresponding to the first-level candidate reference action data is sufficiently small. Therefore, when the value of the comprehensive difference degree is less than the first preset value, the distance between the user action data and the first-level candidate reference action data may be proved to be relatively small (that is, the similarity is very high), the user action data may be considered to include target action data corresponding to the first-level candidate reference action data. At this time, the first-level candidate reference action data may be determined to be the second-level candidate reference action data. When the comprehensive difference degree value is greater than the first preset value, the similarity between the user action data and the first-level candidate reference action data may be proved to be very low, and the user action data may be determined to not include the target action data corresponding to the reference action.

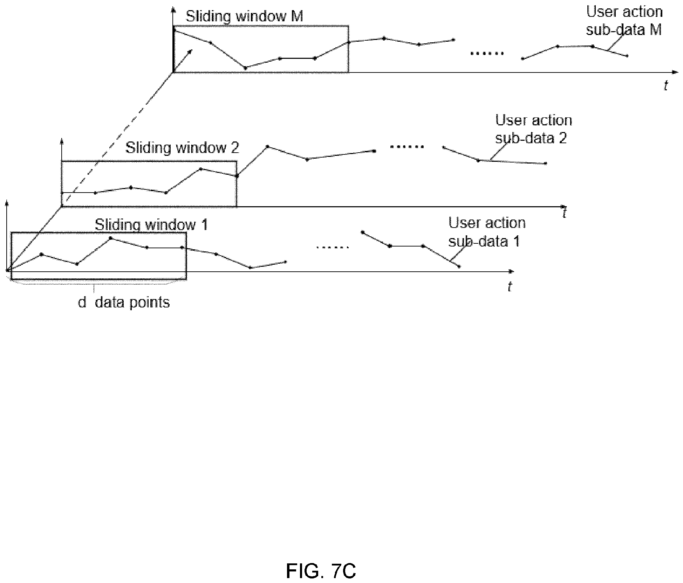

The above illustrates a method for determining the difference degree between a piece of user action sub-data and the corresponding action identification sub-data. FIG. 7C is a schematic diagram illustrating a process for determining a comprehensive difference degree when user action data includes a plurality of pieces of user action sub-data according to some embodiments of the present disclosure.

If action data of a certain action includes data for measuring M parameters, and M is an integer greater than 1, the action data of the action includes M pieces of parallel measurement sub-data. Therefore, the user action data may include M pieces of user action sub-data. The first-level candidate reference action data may also include M pieces of first-level candidate reference action sub-data. Each piece of the first-level candidate reference action sub-data corresponds to local action data in the overall action data obtained by a parameter measurement and includes at least a segment of independent action identification sub-data. All the action identification sub-data together constitute the action identification data of the reference action.

When the first-level candidate reference action sub-data corresponding to a certain piece of user action sub-data includes a segment of action identification sub-data, the comparison sub-result between the user action sub-data and the first-level candidate reference action sub-data may be obtained based on the following operations. The processing device 110 and/or the mobile device 140 may select a segment of the user action data from each of the M pieces of user action sub-data using a sliding window, and sequentially compare the data segments with the corresponding M segments of action identification sub-data in the M pieces of reference action sub-data to determine difference degrees, so that the comparison sub-results may be obtained. Then, the M comparison sub-results may be weighted and summed to obtain a comprehensive difference degree, and whether the user action data includes the target action may be determined based on the comprehensive difference degree and the first preset value. For each piece of the M pieces of user action sub-data, the processing device 110 and/or the mobile device 140 may obtain the difference degree between the user action sub-data and the action identification sub-data based on the method described above.

Specifically, for each piece of the M pieces of user action sub-data, the processing device 110 and/or the mobile device 140 may collect a user action sub-data segment by a sliding window. The sliding windows corresponding to the M pieces of the user action sub-data may be linked or operated independently when sliding. Each sliding window may have the same width, that is, the user action sub-data segment corresponding to each sliding window may uniformly include d (d is an integer greater than 1) data points, and as described above, each data point of the d data points corresponds to a timestamp. Of course, the widths of different sliding windows may also be different, and the amount of data included in the user action sub-data segment in each sliding window may also be different. The processing device 110 and/or the mobile device 140 may determine the overall distance between each data point of the user action sub-data segment and each data point of the action identification sub-data according to the method described above. The processing device 110 and/or the mobile device 140 may obtain the minimum regularization cost of the overall distance through the above method, and then determine the difference degree between the user action sub-data segment and the action identification sub-data. Since there are M pieces of user action sub-data, according to the above method, the processing device 110 and/or the mobile device 140 may obtain M difference degrees in total. Finally, the processing device 110 and/or the mobile device 140 may perform weighted summation on the M comparison sub-results to obtain a comprehensive difference degree.

In some embodiments of the present disclosure, after obtaining the value of the comprehensive difference degree, the processing device 110 and/or the mobile device 140 may directly determine whether the user action data includes the first-level candidate reference action data. For example, the processing device 110 and/or the mobile device 140 may set a first preset value for the action identification data, and when the value of the comprehensive difference degree is greater than the first preset value, the processing device 110 and/or the mobile device 140 may slide each of the M sliding windows to the next data segment at the preset step size, and then the comparison may be repeated. If the comprehensive difference degree value is less than the first preset value, the processing device 110 and/or the mobile device 140 may consider that the set of user action sub-data may include the first-level candidate reference action data, thereby the above-mentioned loop may be ended.

When the first-level candidate reference action sub-data corresponding to a piece of user action sub-data includes a plurality of segments (e.g., p segments, p is an integer greater than 1) of action identification sub-data, a comparison result between the user action sub-data and the first-level candidate reference action sub-data may be obtained based on the following method. For each piece of the M pieces of user action sub-data, the processing device 110 and/or the mobile device 140 may sample a user action sub-data segment from each piece of the user action sub-data through the sliding window. The sliding windows corresponding to the M pieces of the user action sub-data may be linked or operated independently when sliding. Each sliding window may have the same width, that is, the user action sub-data segment corresponding to each sliding window may uniformly include d (d is an integer greater than 1) data points, and as described above, each data point of the d data points corresponds to a timestamp. Of course, the widths of different sliding windows may also be different, and the amount of data included in the user action sub-data segment in each sliding window may also be different. The processing device 110 and/or the mobile device 140 may calculate the p integral distances between the user action sub-data segment and the p segments of action identification sub-data respectively based on the method described above. The processing device 110 and/or the mobile device 140 may determine the minimum regularization cost of the p overall distances by using the above method as a comparison sub-result between the user action sub-data segment and the action identification sub-data. Since there are M pieces of user action sub-data, the processing device 110 and/or the mobile device 140 may obtain M comparison sub-results in total based on the above method. Finally, the processing device 110 and/or the mobile device 140 may perform weighted summation on the M comparison sub-results to obtain the comprehensive difference degree value.

In some embodiments of the present disclosure, after obtaining the value of the comprehensive difference degree, the processing device 110 and/or the mobile device 140 may directly determine whether the set of user action sub-data includes the first-level candidate reference action data. For example, the processing device 110 and/or the mobile device 140 may set a first preset value for the action identification data, and when the comprehensive difference degree value is greater than the first preset value, the processing device 110 and/or the mobile device 140 may slide each of the M sliding windows to the next data segment at the preset step size, and then the comparison may be repeated. If the value of the comprehensive difference degrees is less than the first preset value, the processing device 110 and/or the mobile device 140 may determine that the first-level candidate reference action data is the second-level candidate reference action data, thereby the above-mentioned loop may be ended.

In some embodiments, after determining the second-level candidate reference action data, the processing device 110 and/or the mobile device 140 may further confirm whether the user action includes the reference action corresponding to the second-level candidate reference action data.

In some embodiments of the present disclosure, a second preset value may also be set, and the second preset value may be a preset value related to the probability. Assuming that through the above process, the processing device 110 and/or the mobile device 140 may finally determine that N (N is an integer greater than 1) sets of first-level candidate reference action data are the second-level candidate reference action data. Specifically, N distances (comprehensive comparison results) between the user action data and the N sets of second-level candidate reference action data may be calculated respectively through comparison, and then N probability values may be calculated respectively through the N distances, respectively. The maximum value of the N probability values may be compared with the second preset value, and whether the user action data includes the second-level candidate reference action data corresponding to the maximum probability value may be determined. The probability that the user action data includes the target action corresponding to the i-th second-level candidate reference action data may be expressed as:

$$\frac{1 - {D_i}\big/{\sum_j D_j}}{\sum_i \left(1 - {D_i}\big/{\sum_j D_j}\right)}$$

where $D_j$ denotes the distance (e.g., the aforementioned comprehensive regularization cost or comprehensive difference degree) between the user action data and the j-th second-level candidate reference action data. The smaller the distance value between the user action data and the second-level candidate reference action data is, the higher the probability that the user action data includes the target action corresponding to the second-level candidate reference action data may be. For example, by comparing the user action data with three second-level candidate reference action data (that is, assuming N=3, the numbers of the three second-level candidate reference action data are 1, 2, and 3, respectively), that a distance between the user action data and the second-level candidate reference action data 1 is D1 may be obtained, a distance between the user action data and the second-level candidate reference action data 2 is D2 may be obtained, and a distance between the user action data and the second-level candidate reference action data 3 is D3 may be obtained. The smaller the distance value between the user action data and the second-level candidate reference action data is, the higher the probability that the user action data includes the target action corresponding to the second-level candidate reference action data may be. For example, the probability that the user action data includes the target action corresponding to the second-level candidate reference action data 1 may be determined as follows:

$$\frac{1-D_1/_{D_1+D_2+D_3}}{\left(1-D_1/_{(D_1+D_2+D_3)}\right)+\left(1-D_2/_{(D_1+D_2+D_3)}\right)+\left(1-D_3/_{(D_1+D_2+D_3)}\right)}.$$

The probability that the user action data includes the target action corresponding to the reference action data 2 may be determined as follows:

$$\frac{1-D_2/_{D_1+D_2+D_3}}{\left(1-D_1/_{(D_1+D_2+D_3)}\right)+\left(1-D_2/_{(D_1+D_2+D_3)}\right)+\left(1-D_3/_{(D_1+D_2+D_3)}\right)}.$$

The probability that the user action data includes the target action corresponding to the reference action data 3 may be determined as follows:

$$\frac{1-D_3/_{D_1+D_2+D_3}}{\left(1-D_1/_{(D_1+D_2+D_3)}\right)+\left(1-D_2/_{(D_1+D_2+D_3)}\right)+\left(1-D_3/_{(D_1+D_2+D_3)}\right)}.$$

At this time, the calculated maximum value of the three probability values may be compared with the second preset value. When the maximum probability value is greater than the second preset value, it is determined that the user action data includes the target action corresponding to the second-level candidate reference action data n, and the second-level candidate reference action data n is the second-level candidate reference action data corresponding to the maximum probability value. When the maximum probability value is less than the second-level preset value, it is determined that the user action data that does not include the target action corresponding to the reference action database.

In operation 530, after determining the target action, a content related to the target action may be sent to the user.

Specifically, after the user's action is identified, the action of the user's motion may be monitored, and the monitored information may be sent to the user. Monitoring the action of the user's motion may include monitoring the information related to the user's action. In some embodiments, the information related to the user's action may include one or more of a user action type, an action quantity, the action quality (e.g., whether the user action meets a standard), an action time, or the like. The action type may refer to the fitness action that the user takes when exercising. In some embodiments, the action type may include, but is not limited to, one or more of seated chest clamping, squats, deadlifts, planks, running, swimming, or the like. The action quantity may refer to the count of actions performed during the user's motion. For example, the user may perform 10 seated chest clamping during the user's motion, and the 10 times is the action quantity. The action quality may refer to a standard degree of the fitness action performed by the user relative to a standard fitness action. For example, when the user performs a squat action, the processing device 110 may determine the action type of the user's action based on the feature information of an action signal (the EMG signal and the gesture signal) corresponding to a specific muscle position (the gluteus maximus, the quadricep, etc.), and determine the action quality of the user's squat action based on an action signal of the standard squat action. The action time may refer to a time corresponding to each of one or more action types of the user or a total time of the motion process.

To sum up, the method and system 100 for determining a target action provided by the present disclosure may obtain action data during the user's motion, and then the action data may be compared with reference action data marked with an action content, so that whether the user's motion includes a target action that is the same as the reference action may be identified. The method and system may perform target action identification on the user action data without knowing (it is not known whether the user has performed the action of the annotation type and when the action of the annotation type has been performed) what action the user has performed, and send the content related to the target action to the user after determining the target action. Through the above technical solutions, the present methods and systems have higher intelligence than traditional methods and systems, and improve user experience.

Having thus described the basic concepts, it may be rather apparent to those skilled in the art after reading this detailed disclosure that the foregoing detailed disclosure is intended to be presented by way of example only and is not limiting. Various alterations, improvements, and modifications may occur and are intended to those skilled in the art, though not expressly stated herein. These alterations, improvements, and modifications are intended to be suggested by this disclosure.

Moreover, certain terminology has been configured to describe embodiments of the present disclosure. For example, the terms "one embodiment," "an embodiment," and/or "some embodiments" mean that a particular feature, structure, or characteristic described in connection with the embodiment is included in at least one embodiment of the present disclosure. Therefore, it is emphasized and should be appreciated that two or more references to "an embodiment," "one embodiment," or "an alternative embodiment" in various portions of this specification are not necessarily all referring to the same embodiment. Furthermore, the particular features, structures or characteristics may be combined as suitable in one or more embodiments of the present disclosure.

**Claims**

1. A method for identifying a user action, comprising:

   obtaining (510) user action data collected from a plurality of measurement positions on a user, the user action data corresponding to an unknown user action;
   identifying (520), based on at least one set of target reference action data, that the user action includes a target action when obtaining the user action data, the at least one set of target reference action data corresponding to the target action, wherein the identifying that the user action includes a target action comprises:

      obtaining a plurality of sets of candidate reference action data, wherein each set of candidate reference action data corresponds to at least one reference action;
      performing a two-level screening operation on the plurality of sets of candidate reference action data based on the user action data, the two-level screening operation including a combination of a difference degree-based screening operation and a probability-based screening operation, including:

         obtaining a plurality of sets of reference action data, wherein each set of reference action data corresponds to at least one reference action;
         selecting (521) each set of reference action data in turn from the plurality of sets of reference action data as candidate reference action data;
         determining (522) at least one difference degree by comparing at least one segment of action identification sub-data of the candidate reference action data with the corresponding user action sub-data segment by segment; and
         determining a comprehensive difference degree by weighting and summing the at least one difference degree;
         selecting (523, 524) N pieces of second-level candidate reference action data from the plurality of sets of reference action data, a value of the comprehensive difference degree of the second-level candidate reference action data being less than a first preset value, and N being an integer greater than 1;
         calculating (525) N distances between the user action data and the N pieces of second-level candidate reference action data respectively;
         calculating (526) N probability values based on the N distances respectively;
         selecting (527) the second-level candidate reference action data whose probability value is greater than a second preset value as the target reference action data; and
         determining (528) a reference action corresponding to the target reference action data as the target action; and

      determining that the user action includes the target action based on a result of the two-level screening operation; and

   sending (530) information related to the target action to the user.

2. The method of claim 1, wherein,

   each set of reference action data includes M pieces of reference action sub-data, each piece of the reference action sub-data includes at least one segment of action identification sub-data, and M is an integer greater than 1;
   action identification sub-data of the M pieces of reference action sub-data form integral action identification data, and each segment of action identification sub-data corresponds to at least a portion of the reference action on at least one measurement position of the plurality of measurement positions.

3. The method of claim 1 or claim 2, wherein the determining at least one difference degree by comparing at least one segment of action identification sub-data of the candidate reference action data with the corresponding user action sub-data segment by segment comprises:

   selecting a sliding window with a preset length on each piece of the action identification sub-data, the sliding window including a data segment of the user action data collected in a preset time interval; and
for the sliding window at a current moment, determining the difference degree between the data segment and the corresponding action identification sub-data.

4. The method of claim 3, wherein the identifying that the user action includes the target action further comprises:

determining that a value of the comprehensive difference degree is greater than a first preset value; and
sliding the sliding window to a next data segment with a preset step size, and repeating the comparison.

5. The method of claim 4, wherein a data collection time length corresponding to the data segment in the sliding window is negatively correlated with a user action speed.

6. The method of claim 5, wherein the preset step size satisfies one or more following conditions:

the preset step size is positively correlated with a magnitude of a value of the comprehensive difference degree at a previous moment; and
the preset step size is positively correlated with a variation trend of the value of the comprehensive difference degree.

7. The method of any one of claims 3-6, wherein the data segment comprises a plurality of user action data points; and the determining at least one difference degree by comparing at least one segment of action identification sub-data of the candidate reference action data with the corresponding user action sub-data segment by segment comprises:

selecting a target comparison data interval from the action identification sub-data, wherein the target comparison data interval includes a plurality of identification data points,
adjusting the data segment according to a plurality of scales to obtain a plurality of adjusted data segments,
determining a difference degree between the action identification sub-data and each adjusted data segment of the plurality of adjusted data segments respectively, and
determining a minimum difference degree among the difference degrees between the action identification sub-data and the plurality of adjusted data segments.

8. The method of any one of claims 3-6, wherein the determining at least one difference degree by comparing at least one segment of action identification sub-data of the candidate reference action data with the corresponding user action sub-data segment by segment comprises:

determining a distance matrix $[D_{ij}]$, wherein $D_{ij}$ denotes a distance between an i-th data point of a target comparison data interval and a j-th data point of the data segment;
determining a shortest distance path of the distance matrix, wherein the shortest distance path satisfies:

a start point of the shortest distance path being in the first line of the $[D_{ij}]$,
two adjacent points on the shortest distance path being adjacent in the distance matrix,
a next point on the shortest distance path being to the right, below or right below a previous point,
an end point of the shortest distance path being in a last line of the $[D_{ij}]$, and
the shortest distance path having a smallest regularization cost, wherein the regularization cost is determined by distances of points on the corresponding shortest distance path of the distance matrix; and
the difference degree being related to the regularization cost.

9. The method of claim 8, wherein if the first data point of the data segment is determined to be a data point where the user action starts, the start point of the shortest distance path is a distance $D_{11}$ between the first point of the data segment and the first point of the target comparison data interval.

10. The method of claim 8 or claim 9, wherein if the last data point of the data segment is determined to be the data point where the user action ends, the end point of the shortest distance path is a distance $D_{mn}$ between the last point of the data segment and the last point of the target comparison data interval.

11. A system for identifying a user action, comprising:

an acquisition module (210) configured to obtain user action data collected from a plurality of measurement positions on a user, the user action data corresponding to an unknown user action;
a processing module (220) configured to identify, based on at least one set of target reference action data, that the user action includes a target action when obtaining the user action data, the at least one set of target reference action data corresponding to the target action, wherein to identify, based on at least one set of target reference action data, that the user action includes the target action when obtaining the user action data, the processing module is configured to:

obtain a plurality of sets of candidate reference action data, wherein each set of candidate reference action data corresponds to at least one reference action;
perform a two-level screening operation on the plurality of sets of candidate reference action data based on the user action data, the two-level screening operation including a combination of a difference degree-based screening operation and a probability-based screening operation, including:

obtaining a plurality of sets of reference action data, wherein each set of reference action data corresponds to at least one reference action;
selecting (521) each set of reference action data in turn from the plurality of sets of reference action data as candidate reference action data;
determining (522) at least one difference degree by comparing at least one segment of action identification sub-data of the candidate reference action data with the corresponding user action sub-data segment by segment; and
determining a comprehensive difference degree by weighting and summing the at least one difference degree;
selecting (523, 524) N pieces of second-level candidate reference action data from the plurality of sets of reference action data, a value of the comprehensive difference degree of the second-level candidate reference action data being less than a first preset value, and N being an integer greater than 1;
calculating (525) N distances between the user action data and the N pieces of second-level candidate reference action data respectively;
calculating (526) N probability values based on the N distances respectively;
selecting (527) the second-level candidate reference action data whose probability value is greater than a second preset value as the target reference action data; and
determining (528) a reference action corresponding to the target reference action data as the target action; and

determine that the user action includes the target action based on a result of the two-level screening operation; and
a communication module (240) configured to send information related to the target action to the user.

12. A non-transitory computer readable medium, comprising at least one set of instructions for identifying a user action, wherein when executed by at least one processor of a computing device, the at least one set of instructions direct the at least one processor to perform operations including:

obtaining user action data collected from a plurality of measurement positions on a user, the user action data corresponding to an unknown user action;
identifying, based on at least one set of target reference action data, that the user action includes a target action when obtaining the user action data, the at least one set of target reference action data corresponding to the target action, wherein the identifying that the user action includes a target action comprises:

obtaining a plurality of sets of candidate reference action data, wherein each set of candidate reference action data corresponds to at least one reference action;
performing a two-level screening operation on the plurality of sets of candidate reference action data based on the user action data, the two-level screening operation including a combination of a difference degree-based screening operation and a probability-based screening operation, including:

obtaining a plurality of sets of reference action data, wherein each set of reference action data corresponds to at least one reference action;
selecting (521) each set of reference action data in turn from the plurality of sets of reference action data as candidate reference action data;
determining (522) at least one difference degree by comparing at least one segment of action identification sub-data of the candidate reference action data with the corresponding user action sub-data segment by segment; and

determining a comprehensive difference degree by weighting and summing the at least one difference degree;

selecting (523, 524) N pieces of second-level candidate reference action data from the plurality of sets of reference action data, a value of the comprehensive difference degree of the second-level candidate reference action data being less than a first preset value, and N being an integer greater than 1;

calculating (525) N distances between the user action data and the N pieces of second-level candidate reference action data respectively;

calculating (526) N probability values based on the N distances respectively;

selecting (527) the second-level candidate reference action data whose probability value is greater than a second preset value as the target reference action data; and

determining (528) a reference action corresponding to the target reference action data as the target action; and

determining that the user action includes the target action based on a result of the two-level screening operation; and

sending (530) information related to the target action to the user.

## Patentansprüche

1. Verfahren zum Identifizieren einer Benutzeraktion, umfassend:

Erhalten (510) von Benutzeraktionsdaten, die an einer Vielzahl von Messpositionen an einem Benutzer erfasst wurden, wobei die Benutzeraktionsdaten einer unbekannten Benutzeraktion entsprechen;

Identifizieren (520), basierend auf mindestens einem Satz von Zielreferenzaktionsdaten, dass die Benutzeraktion eine Zielaktion beinhaltet, wenn die Benutzeraktionsdaten erhalten werden, wobei der mindestens eine Satz von Zielreferenzaktionsdaten der Zielaktion entspricht, wobei das Identifizieren, dass die Benutzeraktion eine Zielaktion beinhaltet, umfasst:

Erhalten einer Vielzahl von Sätzen von Kandidaten-Referenzaktionsdaten, wobei jeder Satz von Kandidaten-Referenzaktionsdaten mindestens einer Referenzaktion entspricht;

Durchführen einer zweistufigen Screening-Operation an der Vielzahl von Sätzen von Kandidaten-Referenzaktionsdaten basierend auf den Benutzeraktionsdaten, wobei die zweistufige Screening-Operation eine Kombination aus einer auf dem Differenzgrad basierenden Screening-Operation und einer auf der Wahrscheinlichkeit basierenden Screening-Operation beinhaltet, die beinhaltet:

Erhalten einer Vielzahl von Sätzen von Referenzaktionsdaten, wobei jeder Satz von Referenzaktionsdaten mindestens einer Referenzaktion entspricht;

Auswählen (521) jedes Satzes von Referenzaktionsdaten abwechselnd aus der Vielzahl von Sätzen von Referenzaktionsdaten als Kandidaten-Referenzaktionsdaten;

Bestimmen (522) mindestens eines Differenzgrads durch segmentweises Vergleichen mindestens eines Segments von Aktionsidentifikations-Teildaten der Kandidaten-Referenzaktionsdaten mit den entsprechenden Benutzeraktions-Teildaten; und

Bestimmen eines umfassenden Differenzgrads durch Gewichten und Summieren des mindestens einen Differenzgrads;

Auswählen (523, 524) von N Teilen von Kandidaten-Referenzaktionsdaten zweiter Stufe aus der Vielzahl von Sätzen von Referenzaktionsdaten, wobei ein Wert des umfassenden Differenzgrads der Kandidaten-Referenzaktionsdaten zweiter Stufe kleiner als ein erster voreingestellter Wert ist und N eine ganze Zahl größer als 1 ist;

Berechnen (525) von jeweils N Distanzen zwischen den Benutzeraktionsdaten und den N Teilen der Kandidaten-Referenzaktionsdaten der zweiten Stufe;

Berechnen (526) von jeweils N Wahrscheinlichkeitswerten basierend auf den N Distanzen;

Auswählen (527) der Kandidaten-Referenzaktionsdaten der zweiten Stufe, deren Wahrscheinlichkeitswert größer als ein zweiter voreingestellter Wert ist, als Zielreferenzaktionsdaten; und

Bestimmen (528) einer Referenzaktion, die den Zielreferenzaktionsdaten entspricht, als Zielaktion; und

Bestimmen, dass die Benutzeraktion die Zielaktion beinhaltet, basierend auf einem Ergebnis der zweistufigen Screening-Operation; und Senden (530) von Informationen bezüglich der Zielaktion an den Benutzer.

2. Verfahren nach Anspruch 1, wobei

jeder Satz von Referenzaktionsdaten M Teile von Referenzaktions-Teildaten beinhaltet, jedes Teil der Referenzaktions-Teildaten mindestens ein Segment von Aktionsidentifikations-Teildaten beinhaltet, und M eine ganze Zahl größer als 1 ist;
Aktionsidentifikations-Teildaten der M Teile von Referenzaktions-Teildaten integrale Aktionsidentifikationsdaten bilden, und jedes Segment der Aktionsidentifikations-Teildaten mindestens einem Abschnitt der Referenzaktion an mindestens einer Messposition der Vielzahl von Messpositionen entspricht.

3. Verfahren nach Anspruch 1 oder Anspruch 2, wobei das Bestimmen mindestens eines Differenzgrads durch segmentweises Vergleichen mindestens eines Segments von Aktionsidentifikations-Teildaten der Kandidaten-Referenzaktionsdaten mit den entsprechenden Benutzeraktions-Teildaten umfasst:

Auswählen eines Schiebefensters mit einer voreingestellten Länge für jedes Teil der Aktionsidentifikations-Teildaten, wobei das Schiebefenster ein Datensegment der in einem voreingestellten Zeitintervall erfassten Benutzeraktionsdaten beinhaltet; und
für das Schiebefenster zu einem aktuellen Zeitpunkt, Bestimmen des Differenzgrads zwischen dem Datensegment und den entsprechenden Aktionsidentifikations-Teildaten.

4. Verfahren nach Anspruch 3, wobei das Identifizieren, dass die Benutzeraktion die Zielaktion beinhaltet, weiter umfasst:

Bestimmen, dass ein Wert des umfassenden Differenzgrads größer als ein erster voreingestellter Wert ist; und
Verschieben des Schiebefensters mit einer voreingestellten Schrittweite zu einem nächsten Datensegment und Wiederholen des Vergleichs.

5. Verfahren nach Anspruch 4, wobei eine dem Datensegment in dem Schiebefenster entsprechende Datenerfassungszeitdauer negativ mit einer Benutzeraktionsgeschwindigkeit korreliert ist.

6. Verfahren nach Anspruch 5, wobei die voreingestellte Schrittweite eine oder mehrere folgende Bedingungen erfüllt:

die voreingestellte Schrittweite ist mit einer Größe eines Werts des umfassenden Differenzgrads zu einem vorhergehenden Zeitpunkt positiv korreliert; und
die voreingestellte Schrittweite ist mit einer Variationstendenz des Wertes des umfassenden Differenzgrads positiv korreliert.

7. Verfahren nach einem der Ansprüche 3-6, wobei das Datensegment eine Vielzahl von Benutzeraktionsdatenpunkten umfasst; und
das Bestimmen mindestens eines Differenzgrads durch segmentweises Vergleichen mindestens eines Segments von Aktionsidentifikations-Teildaten der Kandidaten-Referenzaktionsdaten mit den entsprechenden Benutzeraktions-Teildaten umfasst:

Auswählen eines Zielvergleichsdatenintervalls aus den Aktionsidentifikations-Teildaten, wobei das Zielvergleichsdatenintervall eine Vielzahl von Identifikationsdatenpunkten beinhaltet,
Anpassen des Datensegments gemäß einer Vielzahl von Skalen, um eine Vielzahl von angepassten Datensegmenten zu erhalten,
Bestimmen jeweils eines Differenzgrads zwischen den Aktionsidentifikations-Teildaten und jedem angepassten Datensegment der Vielzahl angepasster Datensegmente, und
Bestimmen eines minimalen Differenzgrads unter den Differenzgraden zwischen den Aktionsidentifikations-Teildaten und der Vielzahl von angepassten Datensegmenten.

8. Verfahren nach einem der Ansprüche 3-6, wobei das Bestimmen mindestens eines Differenzgrads durch segmentweises Vergleichen mindestens eines Segments von Aktionsidentifikations-Teildaten der Kandidaten-Referenzaktionsdaten mit den entsprechenden Benutzeraktions-Teildaten umfasst:

Bestimmen einer Distanzmatrix $[D_{ij}]$, wobei $D_{ij}$ eine Distanz zwischen einem i-ten Datenpunkt eines Zielvergleichsdatenintervalls und einem j-ten Datenpunkt des Datensegments bezeichnet;

Bestimmen eines Pfads mit der kürzesten Distanz der Distanzmatrix, wobei der Pfad mit der kürzesten Distanz Folgendes erfüllt:

ein Startpunkt des Pfades mit der kürzesten Distanz ist in der ersten Zeile der $[D_{ij}]$,

zwei benachbarte Punkte des Pfades mit der kürzesten Distanz sind in der Distanzmatrix benachbart,

ein nächster Punkt auf dem Pfad mit der kürzesten Distanz liegt rechts, unterhalb oder direkt unter einem vorhergehenden Punkt,

ein Endpunkt des Pfades mit der kürzesten Distanz ist in einer letzten Zeile der $[D_{ij}]$,

der Pfad mit der kürzesten Distanz weist die geringsten Regulierungskosten auf, wobei die Regulierungskosten durch die Distanzen von Punkten auf dem entsprechenden Pfad mit der kürzesten Distanz der Distanzmatrix bestimmt werden; und

der Differenzgrad hängt mit den Regulierungskosten zusammen.

9. Verfahren nach Anspruch 8, wobei, wenn der erste Datenpunkt des Datensegments als ein Datenpunkt bestimmt wird, an dem die Benutzeraktion beginnt, der Startpunkt des Pfads mit der kürzesten Distanz eine Distanz $D_{11}$ zwischen dem ersten Punkt des Datensegments und dem ersten Punkt des Zielvergleichsdatenintervalls ist.

10. Verfahren nach Anspruch 8 oder Anspruch 9, wobei, wenn der letzte Datenpunkt des Datensegments als der Datenpunkt bestimmt wird, an dem die Benutzeraktion endet, der Endpunkt des Pfads mit der kürzesten Distanz eine Distanz $D_{mn}$ zwischen dem letzten Punkt des Datensegments und dem letzten Punkt des Zielvergleichsdatenintervalls ist.

11. System zum Identifizieren einer Benutzeraktion, umfassend:

ein Erfassungsmodul (210), das dazu konfiguriert ist, Benutzeraktionsdaten zu erhalten, die an einer Vielzahl von Messpositionen an einem Benutzer erfasst wurden, wobei die Benutzeraktionsdaten einer unbekannten Benutzeraktion entsprechen;

ein Verarbeitungsmodul (220), das dazu konfiguriert ist, basierend auf mindestens einem Satz von Zielreferenzaktionsdaten zu identifizieren, dass die Benutzeraktion eine Zielaktion beinhaltet, wenn die Benutzeraktionsdaten erhalten werden, wobei der mindestens eine Satz von Zielreferenzaktionsdaten der Zielaktion entspricht, wobei das Verarbeitungsmodul, um basierend auf mindestens einem Satz von Zielreferenzaktionsdaten zu identifizieren, dass die Benutzeraktion die Zielaktion beinhaltet, wenn die Benutzeraktionsdaten erhalten werden, dazu konfiguriert ist:

eine Vielzahl von Sätzen von Kandidaten-Referenzaktionsdaten zu erhalten, wobei jeder Satz von Kandidaten-Referenzaktionsdaten mindestens einer Referenzaktion entspricht;

eine zweistufige Screening-Operation an der Vielzahl von Sätzen von Kandidaten-Referenzaktionsdaten basierend auf den Benutzeraktionsdaten durchzuführen, wobei die zweistufige Screening-Operation eine Kombination aus einer auf dem Differenzgrad basierenden Screening-Operation und einer auf der Wahrscheinlichkeit basierenden Screening-Operation beinhaltet, die beinhaltet:

Erhalten einer Vielzahl von Sätzen von Referenzaktionsdaten, wobei jeder Satz von Referenzaktionsdaten mindestens einer Referenzaktion entspricht;

Auswählen (521) jedes Satzes von Referenzaktionsdaten abwechselnd aus der Vielzahl von Sätzen von Referenzaktionsdaten als Kandidaten-Referenzaktionsdaten;

Bestimmen (522) mindestens eines Differenzgrads durch segmentweises Vergleichen mindestens eines Segments von Aktionsidentifikations-Teildaten der Kandidaten-Referenzaktionsdaten mit den entsprechenden Benutzeraktions-Teildaten; und

Bestimmen eines umfassenden Differenzgrads durch Gewichten und Summieren des mindestens einen Differenzgrads;

Auswählen (523, 524) von N Teilen von Kandidaten-Referenzaktionsdaten zweiter Stufe aus der Vielzahl von Sätzen von Referenzaktionsdaten, wobei ein Wert des umfassenden Differenzgrads der Kandidaten-Referenzaktionsdaten zweiter Stufe kleiner als ein erster voreingestellter Wert ist und N eine ganze Zahl größer als 1 ist;

Berechnen (525) von jeweils N Distanzen zwischen den Benutzeraktionsdaten und den N Teilen der Kandidaten-Referenzaktionsdaten der zweiten Stufe;

Berechnen (526) von jeweils N Wahrscheinlichkeitswerten basierend auf den N Distanzen;

Auswählen (527) der Kandidaten-Referenzaktionsdaten der zweiten Stufe, deren Wahrscheinlichkeitswert größer als ein zweiter voreingestellter Wert ist, als Zielreferenzaktionsdaten; und

Bestimmen (528) einer Referenzaktion, die den Zielreferenzaktionsdaten entspricht, als Zielaktion; und

zu bestimmen, dass die Benutzeraktion die Zielaktion beinhaltet, basierend auf einem Ergebnis der zweistufigen Screening-Operation; und

ein Kommunikationsmodul (240), das dazu konfiguriert ist, dem Benutzer Informationen bezüglich der Zielaktion zu senden.

12. Nichtflüchtiges computerlesbares Medium, das mindestens einen Satz Anweisungen zum Identifizieren einer Benutzeraktion umfasst, wobei der mindestens eine Satz Anweisungen, wenn er von mindestens einem Prozessor einer Rechenvorrichtung ausgeführt wird, den mindestens einen Prozessor anweist, Operationen durchzuführen, die beinhalten:

Erhalten von Benutzeraktionsdaten, die an einer Vielzahl von Messpositionen an einem Benutzer erfasst wurden, wobei die Benutzeraktionsdaten einer unbekannten Benutzeraktion entsprechen;

Identifizieren, basierend auf mindestens einem Satz von Zielreferenzaktionsdaten, dass die Benutzeraktion eine Zielaktion beinhaltet, wenn die Benutzeraktionsdaten erhalten werden, wobei der mindestens eine Satz von Zielreferenzaktionsdaten der Zielaktion entspricht, wobei das Identifizieren, dass die Benutzeraktion eine Zielaktion beinhaltet, umfasst:

Erhalten einer Vielzahl von Sätzen von Kandidaten-Referenzaktionsdaten, wobei jeder Satz von Kandidaten-Referenzaktionsdaten mindestens einer Referenzaktion entspricht;

Durchführen einer zweistufigen Screening-Operation an der Vielzahl von Sätzen von Kandidaten-Referenzaktionsdaten basierend auf den Benutzeraktionsdaten, wobei die zweistufige Screening-Operation eine Kombination aus einer auf dem Differenzgrad basierenden Screening-Operation und einer auf der Wahrscheinlichkeit basierenden Screening-Operation beinhaltet, die beinhaltet:

Erhalten einer Vielzahl von Sätzen von Referenzaktionsdaten, wobei jeder Satz von Referenzaktionsdaten mindestens einer Referenzaktion entspricht;

Auswählen (521) jedes Satzes von Referenzaktionsdaten abwechselnd aus der Vielzahl von Sätzen von Referenzaktionsdaten als Kandidaten-Referenzaktionsdaten;

Bestimmen (522) mindestens eines Differenzgrads durch segmentweises Vergleichen mindestens eines Segments von Aktionsidentifikations-Teildaten der Kandidaten-Referenzaktionsdaten mit den entsprechenden Benutzeraktions-Teildaten; und

Bestimmen eines umfassenden Differenzgrads durch Gewichten und Summieren des mindestens einen Differenzgrads;

Auswählen (523, 524) von N Teilen von Kandidaten-Referenzaktionsdaten zweiter Stufe aus der Vielzahl von Sätzen von Referenzaktionsdaten, wobei ein Wert des umfassenden Differenzgrads der Kandidaten-Referenzaktionsdaten zweiter Stufe kleiner als ein erster voreingestellter Wert ist und N eine ganze Zahl größer als 1 ist;

Berechnen (525) von jeweils N Distanzen zwischen den Benutzeraktionsdaten und den N Teilen der Kandidaten-Referenzaktionsdaten der zweiten Stufe;

Berechnen (526) von jeweils N Wahrscheinlichkeitswerten basierend auf den N Distanzen;

Auswählen (527) der Kandidaten-Referenzaktionsdaten der zweiten Stufe, deren Wahrscheinlichkeitswert größer als ein zweiter voreingestellter Wert ist, als Zielreferenzaktionsdaten; und

Bestimmen (528) einer Referenzaktion, die den Zielreferenzaktionsdaten entspricht, als Zielaktion; und

Bestimmen, dass die Benutzeraktion die Zielaktion beinhaltet, basierend auf einem Ergebnis der zweistufigen Screening-Operation; und

Senden (530) von Informationen bezüglich der Zielaktion an den Benutzer.

## Revendications

1. Procédé d'identification d'une action d'utilisateur, comprenant :

l'obtention (510) de données d'action d'utilisateur collectées à partir d'une pluralité de positions de mesure sur un utilisateur, les données d'action d'utilisateur correspondant à une action d'utilisateur inconnue ;

l'identification (520), sur la base d'au moins un ensemble de données d'action de référence cible, que l'action d'utilisateur inclut une action cible lors de l'obtention des données d'action d'utilisateur, le au moins un ensemble de données d'action de référence cible correspondant à l'action cible, dans lequel l'identification que l'action d'utilisateur inclut une action cible comprend :

l'obtention d'une pluralité d'ensembles de données d'action de référence candidate, dans lequel chaque ensemble de données d'action de référence candidate correspond à au moins une action de référence ;

la réalisation d'une opération de filtrage à deux niveaux sur la pluralité d'ensembles de données d'action de référence candidate sur la base des données d'action d'utilisateur, l'opération de filtrage à deux niveaux incluant une combinaison d'une opération de filtrage basée sur un degré de différence et d'une opération de filtrage basée sur une probabilité, incluant :

l'obtention d'une pluralité d'ensembles de données d'action de référence, dans lequel chaque ensemble de données d'action de référence correspond à au moins une action de référence ;

la sélection (521) tour à tour de chaque ensemble de données d'action de référence parmi la pluralité d'ensembles de données d'action de référence en tant que données d'action de référence candidate ;

la détermination (522) d'au moins un degré de différence en comparant au moins un segment de sous-données d'identification d'action des données d'action de référence candidate aux sous-données d'action d'utilisateur correspondantes segment par segment ; et

la détermination d'un degré de différence global en pondérant et en additionnant le au moins un degré de différence ;

la sélection (523, 524) de N éléments de données d'action de référence candidate de second niveau parmi la pluralité d'ensembles de données d'action de référence, une valeur du degré de différence global des données d'action de référence candidate de second niveau étant inférieure à une première valeur prédéfinie, et N étant un nombre entier supérieur à 1 ;

le calcul (525) de N distances entre les données d'action d'utilisateur et respectivement les N éléments de données d'action de référence candidate de second niveau ;

le calcul (526) de N valeurs de probabilité sur la base des N distances respectivement ;

la sélection (527) des données d'action de référence candidate de second niveau dont une valeur de probabilité est supérieure à une seconde valeur prédéfinie en tant que données d'action de référence cible ; et

la détermination (528) d'une action de référence correspondant aux données d'action de référence cible en tant qu'action cible ; et

la détermination que l'action d'utilisateur inclut l'action cible sur la base d'un résultat de l'opération de filtrage à deux niveaux ; et l'envoi (530) d'informations relatives à l'action cible à l'utilisateur.

2. Procédé selon la revendication 1, dans lequel,

chaque ensemble de données d'action de référence inclut M éléments de sous-données d'action de référence, chaque élément des sous-données d'action de référence inclut au moins un segment de sous-données d'identification d'action, et M est un nombre entier supérieur à 1 ;

des sous-données d'identification d'action des M éléments de sous-données d'action de référence forment des données d'identification d'action intégrales, et chaque segment de sous-données d'identification d'action correspond à au moins une partie de l'action de référence sur au moins une position de mesure de la pluralité de positions de mesure.

3. Procédé selon la revendication 1 ou 2, dans lequel la détermination d'au moins un degré de différence en comparant au moins un segment de sous-données d'identification d'action des données d'action de référence candidate aux sous-données d'action d'utilisateur correspondantes segment par segment comprend :

la sélection d'une fenêtre glissante d'une longueur prédéfinie sur chaque élément des sous-données d'identification d'action, la fenêtre glissante incluant un segment de données des données d'action d'utilisateur collectées dans un intervalle de temps prédéfini ; et

pour la fenêtre glissante à un moment actuel, la détermination du degré de différence entre le segment de données et les sous-données d'identification d'action correspondantes.

4. Procédé selon la revendication 3, dans lequel l'identification que l'action d'utilisateur inclut l'action cible comprend en outre :

> la détermination qu'une valeur du degré de différence global est supérieure à une première valeur prédéfinie ; et
> le glissement de la fenêtre glissante vers un segment de données suivant d'une taille de pas prédéfinie et la répétition de la comparaison.

5. Procédé selon la revendication 4, dans lequel une durée de collecte de données correspondant au segment de données dans la fenêtre glissante est corrélée négativement à une vitesse d'action d'utilisateur.

6. Procédé selon la revendication 5, dans lequel la taille de pas prédéfinie satisfait à une ou plusieurs des conditions suivantes :

> la taille de pas prédéfinie est corrélée positivement à une grandeur d'une valeur du degré de différence global à un moment précédent ; et
> la taille de pas prédéfinie est corrélée positivement à une tendance de variation de la valeur du degré de différence global.

7. Procédé selon l'une quelconque des revendications 3-6, dans lequel le segment de données comprend une pluralité de points de données d'action d'utilisateur ; et
la détermination d'au moins un degré de différence en comparant au moins un segment de sous-données d'identification d'action des données d'action de référence candidate aux sous-données d'action d'utilisateur correspondantes segment par segment comprend :

> la sélection d'un intervalle de données de comparaison cible à partir des sous-données d'identification d'action, dans lequel l'intervalle de données de comparaison cible inclut une pluralité de points de données d'identification,
> l'ajustement du segment de données selon une pluralité d'échelles pour obtenir une pluralité de segments de données ajustés,
> la détermination d'un degré de différence entre les sous-données d'identification d'action et respectivement chaque segment de données ajusté de la pluralité de segments de données ajustés, et
> la détermination d'un degré de différence minimal parmi les degrés de différence entre les sous-données d'identification d'action et la pluralité de segments de données ajustés.

8. Procédé selon l'une quelconque des revendications 3-6, dans lequel la détermination d'au moins un degré de différence en comparant au moins un segment de sous-données d'identification d'action des données d'action de référence candidate aux sous-données d'action d'utilisateur correspondantes segment par segment comprend :

> la détermination d'une matrice de distance $[D_{ij}]$, dans lequel $D_{ij}$ désigne une distance entre un i-ème point de données d'un intervalle de données de comparaison cible et un j-ème point de données du segment de données;
> la détermination d'un chemin de distance le plus court de la matrice de distance, dans lequel le chemin de distance le plus court satisfait aux conditions suivantes :
>
> > un point de départ du chemin de distance le plus court est dans la première ligne de la $[D_{ij}]$,
> > deux points adjacents sur le chemin de distance le plus court sont adjacents dans la matrice de distance,
> > un point suivant sur le chemin de distance le plus court est à droite, en dessous ou juste en dessous d'un point précédent,
> > un point de fin du chemin de distance le plus court est dans une dernière ligne de la $[D_{ij}]$, et
> > le chemin de distance le plus court présentant le plus petit coût de régularisation, dans lequel le coût de régularisation est déterminé par des distances de points sur le chemin de distance le plus court correspondant de la matrice de distance ; et
> > le degré de différence est lié au coût de régularisation.

9. Procédé selon la revendication 8, dans lequel, si le premier point de données du segment de données est déterminé comme étant un point de données où l'action d'utilisateur commence, le point de début du chemin de distance le plus court est une distance $D_{11}$ entre le premier point du segment de données et le premier point de l'intervalle de données de comparaison cible.

10. Procédé selon la revendication 8 ou 9, dans lequel, si le dernier point de données du segment de données est déterminé comme étant le point de données où l'action d'utilisateur se termine, le point de fin du chemin de distance le plus court est une distance $D_{mn}$ entre le dernier point du segment de données et le dernier point de l'intervalle de données de comparaison cible.

11. Système pour identifier une action d'utilisateur, comprenant :

un module d'acquisition (210) configuré pour obtenir des données d'action d'utilisateur collectées à partir d'une pluralité de positions de mesure sur un utilisateur, les données d'action d'utilisateur correspondant à une action d'utilisateur inconnue ;
un module de traitement (220) configuré pour identifier, sur la base d'au moins un ensemble de données d'action de référence cible, que l'action d'utilisateur inclut une action cible lors de l'obtention des données d'action d'utilisateur, le au moins un ensemble de données d'action de référence cible correspondant à l'action cible, dans lequel, pour identifier, sur la base d'au moins un ensemble de données d'action de référence cible, que l'action d'utilisateur inclut l'action cible lors de l'obtention des données d'action d'utilisateur, le module de traitement est configuré pour :

obtenir une pluralité d'ensembles de données d'action de référence candidate, dans lequel chaque ensemble de données d'action de référence candidate correspond à au moins une action de référence ;
réaliser une opération de filtrage à deux niveaux sur la pluralité d'ensembles de données d'action de référence candidate sur la base des données d'action d'utilisateur, l'opération de filtrage à deux niveaux incluant une combinaison d'une opération de filtrage basée sur le degré de différence et d'une opération de filtrage basée sur la probabilité, incluant :

l'obtention d'une pluralité d'ensembles de données d'action de référence, dans lequel chaque ensemble de données d'action de référence correspond à au moins une action de référence ;
la sélection (521) tour à tour de chaque ensemble de données d'action de référence parmi la pluralité d'ensembles de données d'action de référence en tant que données d'action de référence candidate ;
la détermination (522) d'au moins un degré de différence en comparant au moins un segment de sous-données d'identification d'action des données d'action de référence candidates aux sous-données d'action d'utilisateur correspondantes segment par segment ; et
la détermination d'un degré de différence global en pondérant et en additionnant le au moins un degré de différence ;
la sélection (523, 524) de N éléments de données d'action de référence candidate de second niveau parmi la pluralité d'ensembles de données d'action de référence, une valeur du degré de différence global des données d'action de référence candidate de second niveau étant inférieure à une première valeur prédéfinie, et N étant un nombre entier supérieur à 1 ;
le calcul (525) de N distances entre les données d'action d'utilisateur et respectivement les N éléments de données d'action de référence candidate de second niveau ;
le calcul (526) de N valeurs de probabilité sur la base des N distances respectivement ;
la sélection (527) des données d'action de référence candidate de second niveau dont une valeur de probabilité est supérieure à une seconde valeur prédéfinie en tant que données d'action de référence cible ; et
la détermination (528) d'une action de référence correspondant aux données d'action de référence cible en tant qu'action cible ; et

déterminer que l'action d'utilisateur inclut l'action cible sur la base d'un résultat de l'opération de filtrage à deux niveaux ; et
un module de communication (240) configuré pour envoyer des informations relatives à l'action cible à l'utilisateur.

12. Support non transitoire lisible par ordinateur, comprenant au moins un ensemble d'instructions pour identifier une action d'utilisateur, dans lequel, lorsqu'il est exécuté par au moins un processeur d'un dispositif informatique, le au moins un ensemble d'instructions ordonne au au moins un processeur de réaliser des opérations incluant :

l'obtention de données d'action d'utilisateur collectées à partir d'une pluralité de positions de mesure sur un utilisateur, les données d'action d'utilisateur correspondant à une action d'utilisateur inconnue ;
l'identification, sur la base d'au moins un ensemble de données d'action de référence cible, que l'action d'utilisateur inclut une action cible lors de l'obtention des données d'action d'utilisateur, le au moins un ensemble de données d'action de référence cible correspondant à l'action cible, dans lequel l'identification que l'action d'utilisateur inclut une action cible comprend :

l'obtention d'une pluralité d'ensembles de données d'action de référence candidates, dans lequel chaque ensemble de données d'action de référence candidates correspond à au moins une action de référence ;
la réalisation d'une opération de filtrage à deux niveaux sur la pluralité d'ensembles de données d'action de référence candidate sur la base des données d'action d'utilisateur, l'opération de filtrage à deux niveaux incluant une combinaison d'une opération de filtrage basée sur le degré de différence et d'une opération de filtrage basée sur la probabilité, incluant :

l'obtention d'une pluralité d'ensembles de données d'action de référence, dans lequel chaque ensemble de données d'action de référence correspond à au moins une action de référence ;
la sélection (521) tour à tour de chaque ensemble de données d'action de référence parmi la pluralité d'ensembles de données d'action de référence en tant que données d'action de référence candidate ;
la détermination (522) d'au moins un degré de différence en comparant au moins un segment de sous-données d'identification d'action des données d'action de référence candidates aux sous-données d'action d'utilisateur correspondantes segment par segment ; et
la détermination d'un degré de différence global en pondérant et en additionnant le au moins un degré de différence ;
la sélection (523, 524) de N éléments de données d'action de référence candidate de second niveau parmi la pluralité d'ensembles de données d'action de référence, une valeur du degré de différence global des données d'action de référence candidate de second niveau étant inférieure à une première valeur prédéfinie, et N étant un nombre entier supérieur à 1 ;
le calcul (525) de N distances entre les données d'action d'utilisateur et respectivement les N éléments de données d'action de référence candidate de second niveau ;
le calcul (526) de N valeurs de probabilité sur la base des N distances respectivement ;
la sélection (527) des données d'action de référence candidate de second niveau dont une valeur de probabilité est supérieure à une seconde valeur prédéfinie en tant que données d'action de référence cible ; et
la détermination (528) d'une action de référence correspondant aux données d'action de référence cible en tant qu'action cible ; et

la détermination que l'action d'utilisateur inclut l'action cible sur la base d'un résultat de l'opération de filtrage à deux niveaux ; et
l'envoi (530) d'informations relatives à l'action cible à l'utilisateur.

FIG. 1

130

FIG. 2

**300**

380

370

360

350

Come form Internet/
Go to Internet

Hard disk

Input/Output
interface

Communication
port

310

320

330

340

Processor

Read only
memory

Random access
memory

FIG. 3

FIG. 4

500

Obtaining user action data during a user's motion — 510

Performing an action identification on the user action data based on one or more sets of candidate reference action data whose content are known — 520

521. Selecting each set of reference action data in turn from the plurality of sets of reference action data as the first-level candidate reference action data

522. Determining a difference degree between the first-level candidate reference action data and the user action data

523. Determining whether the difference degree value is less than a first preset value? — N

Y

524. Determining the first-level candidate reference action data as the second-level candidate reference action data

525. Determining a distance between the user action data and each of the multiple sets of the second-level candidate reference action data

526. Determining a probability that the user action data includes the target action corresponding to the second-level candidate reference action data based on each distance

527. Determining whether the maximum value among the values of the probabilities is greater than a second preset value? — N

Y

528. Determining that the user action includes a reference action corresponding to the second-level candidate reference action data with the highest probability value, and the reference action being the target action

529. Determining that the user action does not include the reference action corresponding to the second-level candidate reference action data

After determining the target action, sending a content related to the target action to the user — 530

FIG. 5

FIG. 6

FIG. 7A

FIG. 7B

Sliding window M

User action
sub-data M

*t*

User action
sub-data 2

Sliding window 2

*t*

Sliding window 1

User action
sub-data 1

d  data points

*t*

FIG. 7C

**REFERENCES CITED IN THE DESCRIPTION**

**Patent documents cited in the description**

- CN 108211309 A
- CN 108209910 A
- CN 110569775 A
- CN 107349594 A